# EUROPEAN PATENT APPLICATION

(11) **EP 4 445 919 A1**
(43) Date of publication of application: **16.10.2024**
(21) Application number: 22903276.8
(22) Date of filing: 30.11.2022
(51) Int. Cl.: A61K 47/68, C07D 307/20, C07K 16/28, A61P 35/00

(54) **ANTIBODY-DRUG CONJUGATE HAVING IMPROVED AFFINITY, AND PREPARATION METHOD THEREFOR AND APPLICATION THEREOF**

(30) Priority: 09.12.2021 CN 202111496135
(71) Applicant: Kunshan Xinyunda Biotech Co., Ltd., Kunshan, Jiangsu 215300 (CN)
(72) Inventor: DING, Hui, Kunshan, Jiangsu 215300 (CN); KE, Tianyi, Kunshan, Jiangsu 215300 (CN); YU, Haiyong, Kunshan, Jiangsu 215300 (CN); XU, Yunlei, Kunshan, Jiangsu 215300 (CN); LAO, Fang, Kunshan, Jiangsu 215300 (CN); LIU, Yan, Kunshan, Jiangsu 215300 (CN); ZHANG, Xidong, Kunshan, Jiangsu 215300 (CN); RONG, Pengfei, Kunshan, Jiangsu 215300 (CN)
(74) Representative: van Dam, Vincent
(86) International application number: PCT/CN2022/135429
(87) International publication number: WO 2023/103854

(57) **Abstract**

An antibody-drug conjugate having improved affinity, and a preparation method therefor and an application thereof. Specifically, provided are an antibody drug conjugate as shown in formula (II), a pharmaceutically acceptable salt thereof, a stereoisomer thereof, or a solvate thereof. The antibody drug conjugate no only obviously improves the affinity of an antibody and a target protein, but also improves the hydrophilicity of an ADC molecule, and further improves the efficacy of tumor inhibition..

## Description

### Technical Field

The present disclosure relates to the field of medicine, and specifically to an antibody-drug conjugate (ADC) capable of binding to a human oncology antigen target and/or providing an anti-tubulin drug activity. The present disclosure further relates to a method and composition useful in the treatment and diagnosis of a cancer that expresses a tumor antigen target and/or is treatable by disrupting tubulin.

### Background Art

MORAb-202 is the first ADC of Eisai, which is the first ADC that the in-house developed anti-cancer agent eribulin of Eisai is conjugated with the humanized IgG1 monoclonal antibody targeting to folate receptor α (FRα), and has the structure as shown below, in which the antibody and the toxin are linked using an enzyme-cleavable linker, and the drug-to-antibody ratio (DAR) is 4.0.

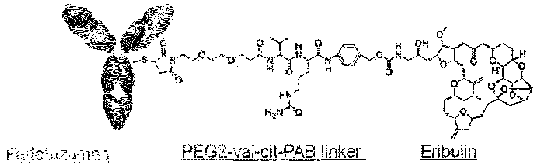

The MORAb-202's payload eribulin (product name: Halaven) and paclitaxel are both tubulin inhibitors, but eribulin is the first halichondrin-type tubulin dynamics inhibitor, which has a novel mechanism of action. Structurally, eribulin is a simplified and synthetic version of halichondrin B. Halichondrin B is a natural product isolated from the sponge *Halichondria okadai.* Eribulin is thought to prevent cell division by inhibiting tubulin dynamics during the growth phase. In cancer cells, this mechanism can lead to prolonged irreversible mitotic arrest, ultimately leading to cell death. Eribulin was approved for marketing in China in January 2020 for the treatment of patients with topically recurrent or metastatic breast cancer who have previously received at least 2 chemotherapy regimens (including anthracyclines and taxanes). Therefore, Eribulin can effectively solve the problem of drug resistance caused by treatment with tubulin inhibitors such as anthracyclines and taxanes.

The linker of MORAb-202 adopts the structure of PEG2-val-cit-PAB. The addition of p-aminobenzyloxycarbonyl (PAB) group is to provide sufficient enzyme cleavage space so that ADC can be self-cleaved in the cell to release eribulin small molecule. The PEG2 structure provides better hydrophilicity for ADC molecule.

### Contents of the present invention

Although MORAb-202 has shown good clinical effects and application prospects, there is still room for improvement, especially in the linker part. As a bridge of ADC drug, the linker is linked to the antibody through a cleavable or non-cleavable linker. The linker needs to be exquisitely designed, requiring both stability and specific cleavability in tumor cells, as well as pharmaceutical convenience, such as good hydrophilicity, suitable DAR value, and maintaining good affinity of the antibody. Therefore, there is still room and demand for optimizing the linker of ADC.

The inventors of the present disclosure creatively discovered that the molecular structure of eribulin carries a primary amine structure, and the primary amine structure is far away from the main structure of eribulin, so that there is a sufficient space. Based on this, the inventors designed a PAB-free linker to directly connect the primary amine structure of eribulin with an amino acid or short peptide (e.g., Vc or Va structure) to form an amide bond; in addition, the inventors of the present disclosure also creatively discovered that the hydrophilicity of the ADC can be improved by using a short chain instead of a long chain after the maleimide group to connect to the amino acid or short peptide (e.g., Vc or Va). By the design based on the "truncation" concept in the two aspects mentioned above, the inventors obtained the linker, linker-eribulin small molecule compound, and ADC of the present disclosure. The inventors of the present disclosure unexpectedly discovered that the ADC constructed by the truncated linker of the present disclosure can not only significantly improve the hydrophilicity of the ADC molecule, but also improve the affinity between the antibody and the target protein, and can also improve the anti-tumor efficacy.

To this end, in the first aspect of the present disclosure, the present disclosure provides a compound represented by Formula (I), a pharmaceutically acceptable salt thereof, a stereoisomer thereof, or a solvate thereof,

L¹-L²-D (I)

wherein:
L¹ is 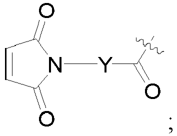
Yis 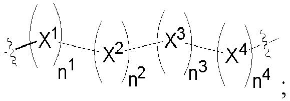
X¹, X², X³ and X⁴ are each independently selected from the group consisting of CH₂, NH, O and S, and at least one of X¹, X², X³ and X⁴ is CH₂;
n¹, n², n³ and n⁴ are each independently selected from the group consisting of 0 and 1, and at least one of n¹, n², n³ and n⁴ is 1;
L² is selected from the group consisting of amino acid residues and a short peptide consisting of 2 to 10 amino acid residues; preferably, the amino end of L² is linked to L¹, and the carbonyl end of L² is linked to D;
D is a drug linked to L² through a chemical bond, and the drug is preferably eribulin or a derivative thereof.

In some embodiments, Y is

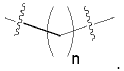

In some embodiments, X¹, X², X³ and X⁴ are all CH₂.

In some embodiments, n¹ is 0, n², n³, and n⁴ are each independently selected from the group consisting of 0 and 1, and at least one of n², n³, and n⁴ is 1.

In some embodiments, n¹ and n² are 1, and n³ and n⁴ are 0.

In some embodiments, n is selected from the group consisting of 1, 2, 3 and 4.

In some embodiments, n is selected from the group consisting of 1, 2 and 3.

In some embodiments, n is 2.

In some embodiments, L² is a short peptide consisting of 2 to 4 amino acid residues, and preferably, the amino acid is selected from the group consisting of glycine, phenylalanine, valine, citrulline, and alanine, more preferably, the amino acid is selected from the group consisting of valine, citrulline, and alanine. In some embodiments, the amino end of L² is linked to L¹, and the carbonyl end of L² is linked to D.

In some embodiments, L² is selected from the group consisting of glycine-glycine-phenylalanine-glycine (GGFG), valine-citrulline (VC), valine-alanine (VA), preferably selected from the group consisting of valine-citrulline (VC), and valine-alanine (VA). In some embodiments, the amino end of L² is linked to L¹, and the carbonyl end of L² is linked to D.

In some embodiments, L² is selected from the group consisting of 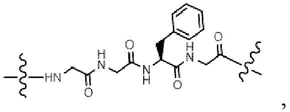 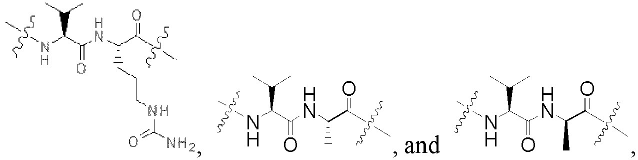 preferably selected from the group consisting of 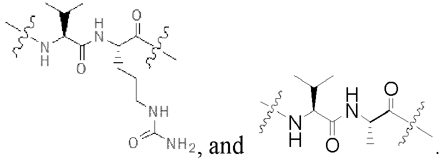 In some embodiments, the amino end of L² is linked to L¹, and the carbonyl end of L² is linked to D.

In some embodiments, D is

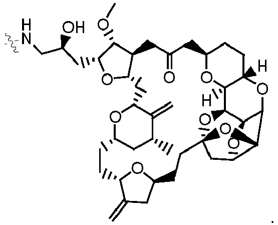

In some embodiments, the linker is stable extracellularly such that the ADC remains intact when present in the extracellular environment but is capable of being cleaved when internalized in a cell, such as a cancer cell. In some embodiments, when the ADC enters a cell expressing an antigen specific for the antibody moiety of the ADC, the eribulin drug moiety is cleaved from the antibody moiety, and the unmodified form of eribulin is cleaved and released. In some embodiments, the linker comprises a cleavable moiety that enables the linker or the antibody moiety to remain bound to eribulin after cleavage.

In some embodiments, the cleavable moiety in the linker is a cleavable peptide moiety. In some embodiments, compared to an ADC comprising alternative cleavable moiety, an ADC comprising a cleavable peptide moiety exhibits a lower aggregation level, an improved antibody: drug ratio, increased target killing of cancer cells, reduced off-target killing of non-cancer cells, and/or a higher drug loading (p). In some embodiments, compared to a non-cleavable linker, the addition of a cleavable moiety increases the cytotoxicity and/or potency. In some embodiments, the increased potency and/or cytotoxicity is shown in cancers that express moderate levels of the antigen targeted by the antibody moiety of the ADC (e.g., moderate FRA expression). In some embodiments, the cleavable peptide moiety is enzyme-cleavable and the linker is an enzyme-cleavable linker. In some embodiments, the enzyme is a cathepsin, and the linker is a cathepsin-cleavable linker. In certain embodiments, compared to alternative cleavage mechanisms, the enzyme-cleavable linker (e.g., cathepsin-cleavable linker) exhibits one or more of the improved properties described above.

In some embodiments, the cleavable peptide moiety of the linker comprises an amino acid unit. In some embodiments, the amino acid unit comprises valine-citrulline (Val-Cit) or valine-alanine (Val-Ala). In some embodiments, compared to an ADC comprising alternative amino acid unit or alternative cleavable moiety, an ADC comprising Val-Cit or Val-Ala exhibits increased stability, reduced off-target cell killing, increased on-target cell killing, lower aggregation level, and/or higher drug loading.

In some embodiments, the linker comprises at least one spacer unit that joins the antibody moiety to the cleavable moiety. In some embodiments, the spacer unit in the linker may comprise at least one maleimidocaproyl (MC) moiety or a truncated fragment thereof, preferably a truncated fragment of MC. In some embodiments, despite the shorter linker length, an ADC comprising a shorter spacer unit (e.g., truncated MC) shows lower aggregation level and better stability and/or efficacy as compared to an ADC comprising a longer spacer unit (e.g., MC or (PEG)₂₋₈).

In some embodiments, the spacer unit of the linker in the antibody conjugate of the present disclosure does not comprise the spacer unit p-aminobenzyloxycarbonyl (PAB) attached to D.

In some embodiments, the spacer unit in the linker is attached to the antibody moiety of the ADC via a maleimide moiety (Mal).

In certain embodiments, the compound represented by L¹-L²-D is selected from the group consisting of: 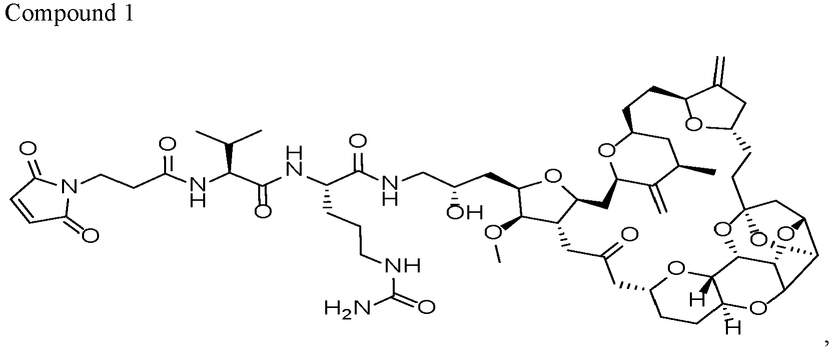 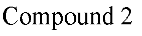 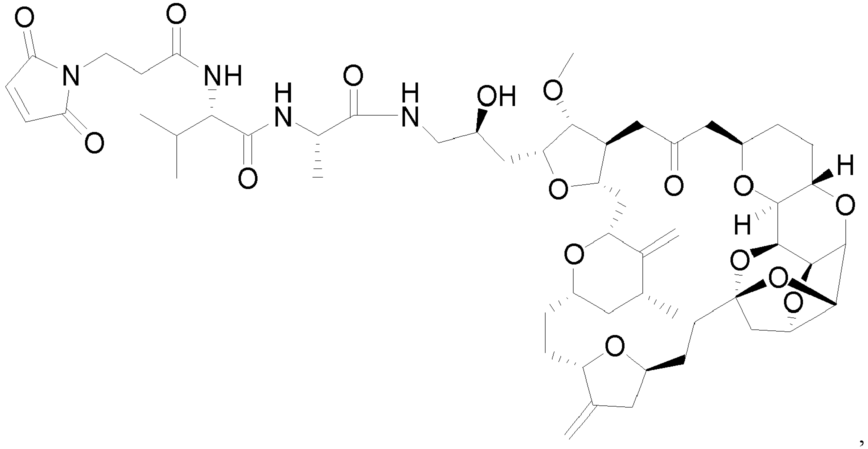 and

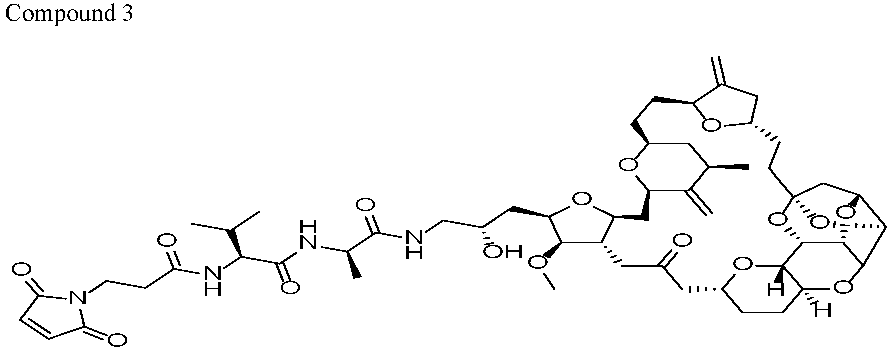

In addition, the following Control Compound 1 and Control Compound 2 are only used as control structures of the compound represented by Formula (I) of the present disclosure, and are not the compounds sought to be protected by the present disclosure. 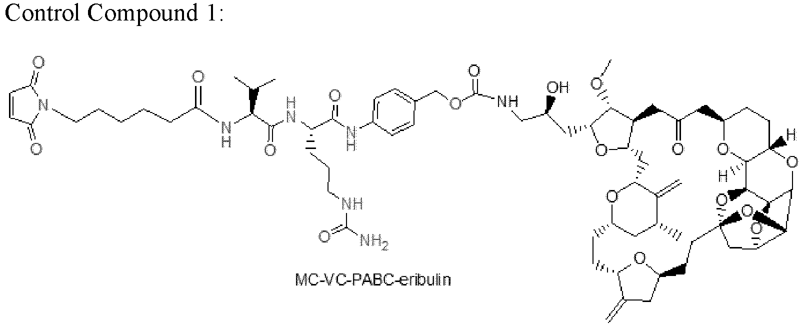 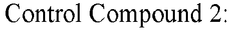 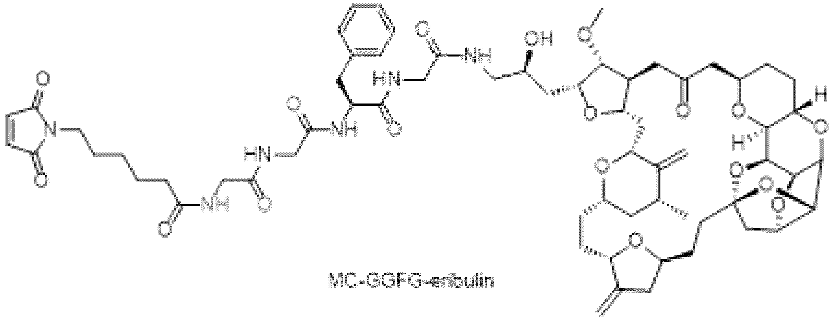 In the second aspect of the present disclosure, the present disclosure provides an antibody-drug conjugate represented by Formula (II), a pharmaceutically acceptable salt thereof, a stereoisomer thereof, or a solvate thereof,

Ab-(L¹-L²-D)ₚ (II)

wherein:
Ab is an antibody or an antigen-binding fragment thereof;
L¹ is 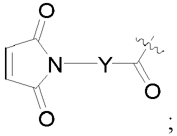
Yis 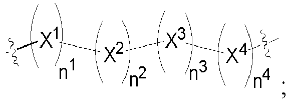
X¹, X², X³ and X⁴ are each independently selected from the group consisting of CH₂, NH, O and S, and at least one of X¹, X², X³ and X⁴ is CH₂;
n¹, n², n³ and n⁴ are each independently selected from the group consisting of 0 and 1, and at least one of n¹, n², n³ and n⁴ is 1;
L² is selected from the group consisting of amino acid residues and a short peptide consisting of 2 to 10 amino acid residues; preferably, the amino end of L² is linked to L¹, and the carbonyl end of L² is linked to D;
D is a drug linked to L² through a chemical bond, and the drug is preferably eribulin or a derivative thereof;
p is any value between 1 and 20.

In some embodiments, Y is

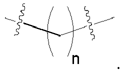

In some embodiments, X¹, X², X³ and X⁴ are all CH₂.

In some embodiments, n¹ is 0, n², n³, and n⁴ are each independently selected from the group consisting of 0 and 1, and at least one of n², n³, and n⁴ is 1.

In some embodiments, n¹ and n² are 1, and n³ and n⁴ are 0.

In some embodiments, n is selected from the group consisting of 1, 2, 3 and 4.

In some embodiments, n is selected from the group consisting of 1, 2 and 3.

In some embodiments, n is 2.

In some embodiments, L² is a short peptide consisting of 2-4 amino acid residues. Preferably, the amino acid is selected from the group consisting of glycine, phenylalanine, valine, citrulline, and alanine, more preferably, the amino acid is selected from the group consisting of valine, citrulline, and alanine. In some embodiments, the amino end of L² is linked to L¹ and the carbonyl end of L² is linked to D.

In some embodiments, L² is selected from the group consisting of glycine-glycine-phenylalanine-glycine (GGFG), valine-citrulline (VC), valine-alanine (VA), preferably selected from the group consisting of valine-citrulline (VC), and valine-alanine (VA). In some embodiments, the amino end of L² is linked to L¹ and the carbonyl end of L² is linked to D.

In some embodiments, L² is selected from the group consisting of 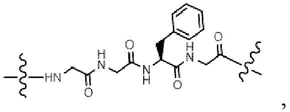 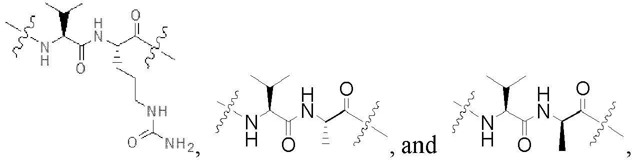 preferably selected from the group consisting of 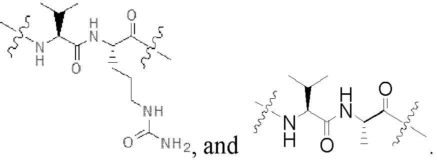 In some embodiments, the amino end of L² is linked to L¹, and the carbonyl end of L² is linked to D.

In some embodiments, D is

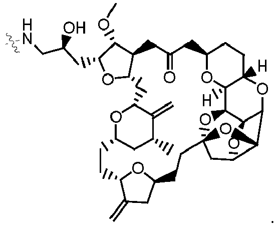

It should be noted that the "antibody-drug conjugate" represented by the above Formula (II) refers to a composition of ADC molecules with the same or different numbers of conjugated drug molecules.

Specifically, the present disclosure provides a composition comprising a plurality of ADC molecules. In certain cases, each ADC in the composition described herein comprises the same number of one or more drug molecules. In other cases, each ADC in the composition described herein comprises a different number of one or more drug molecules.

In the antibody-drug conjugate described herein, each antibody can be conjugated with 1, 2, 3, 4, 5, 6, 7, 8 or more drug molecules, for example, 1 to 5 (e.g., 1, 2, 3, 4, or 5) drug molecules, 5 to 8 (e.g., 5, 6, 7, or 8) drug molecules, or 8 to 12 (e.g., 8, 9, 10, 11 or 12) drug molecules.

The drug-to-antibody ratio (DAR) refers to the number of drug molecules conjugated to the antibody (e.g., p in Formula II). The number of drug molecules contained in the ADC described herein is usually an integer. When the number of drug molecules contained in the ADC described herein (e.g., p in Formula II) is a fraction, the fraction refers to the average number of drug molecules conjugated to each antibody in a composition comprising multiple ADC molecules.

In some embodiments, p is any value between 1 and 10.

In some embodiments, p is any value between 3 and 5.

In some embodiments, p is any value between 3.5 and 4.5, for example, p is 3.5, 3.9, or 4.1.

In some embodiments, the antibody-drug conjugate is selected from the group consisting of the following: 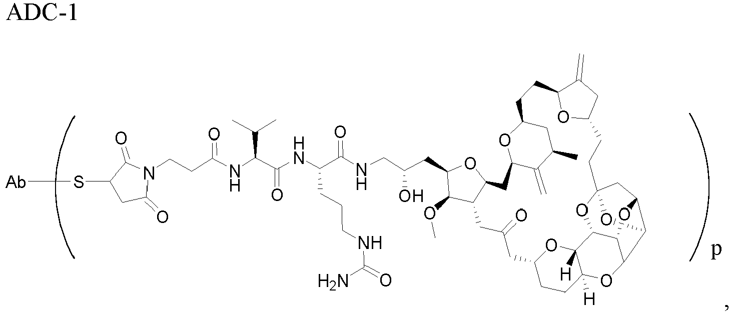 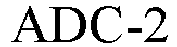 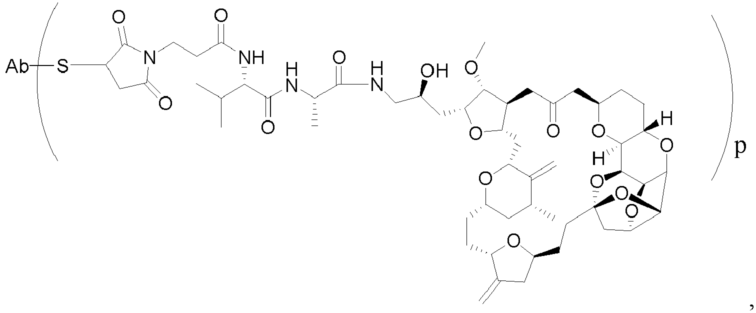 and

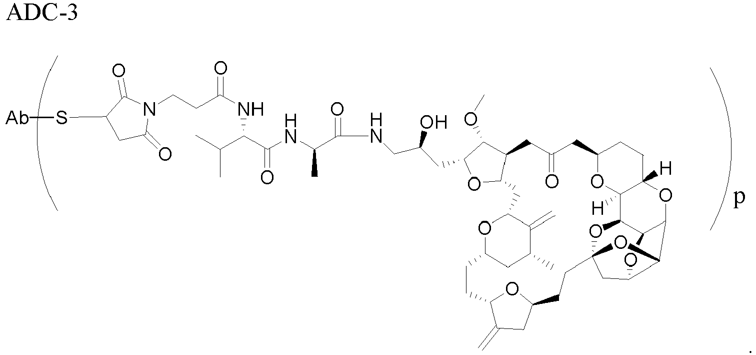

It should be noted that in the above ADC-1, ADC-2 or ADC-3, -S- is not another external sulfur atom, but the -S formed by linking 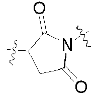 to the sulfhydryl in Ab itself after opening the disulfide bond.

Accordingly, those skilled in the art can understand that in the above Formula (II), after L¹ is linked to Ab, the structural formula of L¹ is 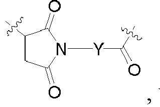 wherein, the carbon end at the left side of 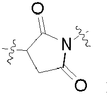 is linked to the sulfhydryl in Ab itself after opening the disulfide bond.

In some embodiments, the antibody in the antibody-drug conjugate (ADC) of the present disclosure is selected from the group consisting of murine antibody, chimeric antibody, humanized antibody, and fully human antibody.

Suitable antibodies may bind to any disease-associated antigens known in the art. When the disease state is a cancer, for example, many antigens expressed by or otherwise associated with tumor cells are known in the art, which include but are not limited to carbonic anhydrase IX, α-fetoprotein (AFP), α-actinin-4, A3, antigen specific for A33 antibody, ART-4, B7, Ba 733, BAGE, BrE3 antigen, CA125, CAMEL, CAP -1, CASP-8/m, CCL19, CCL21, CD1, CD1a, CD2, CD3, CD4, CD5, CD8. CD11A, CD14, CD15, CD16, CD18, CD19, CD20, CD21, CD22, CD23, CD25, CD29, CD30, CD32b, CD33, CD37, CD38, CD40, CD40L, CD44, CD45, CD46, CD52, CD54, CD55, CD59, CD64, CD66a-e, CD67, CD70, CD70L, CD74, CD79a, CD80, CD83, CD95, CD126, CD132, CD133, CD138, CD147, CD154, CDC27, CDK-4/m, CDKN2A, CTLA-4, CXCR4, CXCR7, CXCL12, HIF-1α, colon-specific antigen p (CSAp), CEA (CEACAM5), CEACAM6, c-Met, DAM, EGFR, EGFRvIII, EGP-1 (Trop-2), EGP-2, ELF2-M, Ep-CAM, her2, her3, claudin 18.2, ROR1, ROR2, dll3, marc17, fibroblast growth factor (FGF), Flt-1, Flt-3, folate receptor, G250 antigen, GAGE, gp100, GRO-β, HLA-DR, HM1.24, human chorionic gonadotropin (HCG) and subunit thereof, HER2/neu, HMGB-1, hypoxia-inducible factor (HIF-1), HSP70- 2M, HST-2, Ia, IGF-1R, IFN-γ, IFN-α, IFN-β, IFN-λ, IL-4R, IL-6R, IL-13R, IL-15R, IL-17R, IL- 18R, IL-2, IL-6, IL-8, IL-12, IL-15, IL-17, IL-18, IL-23, IL-25, insulin-like growth factor 1 (IGF-1), IGF -1R, KS1-4, Le-Y, LDR/FUT, macrophage migration inhibitory factor (MIF), MAGE, MAGE-3, MART-1, MART-2, NY-ESO-1, TRAG-3, mCRP, MCP-1, MIP-1A, MIP-1B, MIF, MUC1, MUC2, MUC3, MUC4, MUC5ac, MUC13, MUC16, MUM-1/2, MUM-3, NCA66, NCA95, NCA90, pancreatic cancer mucin, PD-1 receptor, PD-L1 receptor, placental growth factor, p53, PLAGL2, prostatic acid phosphatase, PSA, PRAME, PSMA, PlGF, ILGF, ILGF-1R, IL-6, IL-25, RS5, RANTES, T101, SAGE, S100, survivin, survivin-2B, TAC, TAG-72, tenascin, TRAIL receptor, TNF-α, Tn antigen, Thomson-Friedenreich antigen, tumor necrosis antigen, VEGFR, ED-B fibronectin, WT-1, 17-1A antigen, complement factors C3, C3a, C3b, C5a, C5, angiogenesis markers, bcl-2, bcl-6, Kras, oncogene marker and oncogene product (see, for example, Sensi et al., Clin Cancer Res2006, 12:5023-32; Parmiani et al., J Immunol 2007, 178:1975-79; Novellino et al. Cancer Immunol Immunother 2005, 54:187-207). Preferably, the antibody binds to her2, her3, claudin 18.2, ROR-1, dll-3, mucl, muc-17, EGP-1 (Trop-2).

Exemplary antibodies that may be used include, but are not limited to, hR1 (anti-IGF-1R, U.S. Patent No. 13/688,812), hPAM4 (anti-mucin, U.S. Patent No. 7,282,567), hA20 (anti-CD20, U.S. Patent No. 7,151,164), hA19 (anti-CD19, U.S. Patent No. 7,109,304), hIMMU31 (anti-AFP, U.S. Patent No. 7,300,655), hLL1 (anti-CD74, U.S. Patent No. 7,312,318), hLL2 (anti-CD22, U.S. Patent No. 5,789,554), hMu-9 (anti-CSAp, U.S. Patent No. 7,387,772), hL243 (anti-HLA-DR, U.S. Patent No. 7,612,180), hMN-14 (anti-CEACAM5, U.S. Patent No. 6,676,924), hMN-15 (anti-CEACAM6, U.S. Patent No. 8,287,865), hRS7 (anti-EGP-1, U.S. Patent No. 7,238,785), hMN-3 (anti-CEACAM6, U.S. Patent No. 7,541,440), Ab124 and Ab125 (anti-CXCR4, U.S. Patent No. 7,138,496), the section of examples of each cited patent or application is incorporated herein by reference. More preferably, the antibody is IMMU-31 (anti-AFP), hRS7 (anti-Trop-2), hMN-14 (anti-CEACAM5), hMN-3 (anti-CEACAM6), hMN-15 (anti-CEACAM6), hLL1 (anti-CD74), hLL2 (anti-CD22), hL243 or IMMU-114 (anti-HLA-DR), hA19 (anti-CD19) or hA20 (anti-CD20). As used herein, the terms epratuzumab and hLL2 are interchangeable, as are the terms veltuzumab and hA20, hL243g4P, hL243γ4P, and IMMU-114. In a most preferred embodiment, the antibody is an anti-Trop-2 antibody such as hRS7; an anti-ROR1 antibody such as 99961 (Chinese Patent No. 104662044); an anti-HER-3 antibody such as patritumab (Chinese Patent No. 102174105); an anti-HER2 antibody Herceptin (Trastuzumab), pertuzumab.

Applicable alternative antibodies include, but are not limited to, abciximab (anti-glycoprotein IIb/IIIa), alemtuzumab (anti-CD52), bevacizumab (anti-VEGF), cetuximab (anti-EGFR), gemtuzumab (anti-CD33), ibritumomab (anti-CD20), panitumumab (anti-EGFR), rituximab (anti-CD20), tositumomab (anti-CD20), trastuzumab (anti-ErbB2), lambrolizumab (anti-PD1 receptor), atezolizumab (anti-PD-L1), MEDI4736 (anti-PD-L1), nivolumab (anti-PD-1 receptor), ipilimumab (anti-CTLA-4), abagovomab (anti-CA-125), adecatumumab (anti-EpCAM), atlizumab (anti-IL-6 receptor), benralizumab (anti-CD125), obinutuzumab (GA101, anti-CD20), CC49 (anti-TAG-72), AB-PG1-XG1-026 (anti-PSMA, U.S. Pat. Application 11/983,372, deposited as ATCC PTA-4405 and PTA-4406), D2/B (anti-PSMA, WO 2009/130575), tocilizumab (anti-IL-6 receptor), basiliximab (anti-CD25), daclizumab (anti-CD25), efalizumab (anti-CD11a), GA101 (anti-CD20; GlycartRoche), muromonab-CD3 (anti-CD3 receptor), natalizumab (anti-α4 integrin), omalizumab (anti-IgE); anti-TNF-α antibody such as CDP571 (Ofei et al., 2011, Diabetes 45:881-85), MTNFAI, M2TNFAI, M3TNFAI, M3TNFABI, M302B, M303 (ThermoScientific, Rockford, IL), infliximab (Centocor, Malvern, PA), certolizumab pegol (UCB, Brussels, Belgium), anti-CD40L (UCB, Brussels, Belgium), adalimumab (Abbott, Abbott Park, IL), Benlysta (Human Genome Sciences); antibody for treatment of Alzheimer's disease, such as Alz 50 (Ksiezak-Reding et al., 1987, J Biol Chem 263:7943-47), gantenerumab, solanezumab and infliximab; antifibrin antibody, such as 59D8, T2G1s, MH1; anti-CD38 antibody, such as MOR03087 (MorphoSys AG), MOR202 (Celgene), HuMax-CD38 (Genmab), or daratumumab (Johnson & Johnson); anti-HIV antibody, such as P4/D10 (U.S. Patent 8,333,971), Ab75, Ab76, Ab77 (Paulik et al., 1999, Biochem Pharmacol 58:1781-90), and anti-HIV antibody described and marketed by Polymun (Vienna, Austria), which also described in U.S. Patent 5,831,034, U.S. Patent 5,911,989, and Vcelar et al., AIDS 2007; 21(16):2161-2170, and Joos et al., Antimicrob. Agents Chemother. 2006; 50(5): 1773-9, all these documents are incorporated herein by reference.

In certain embodiments, the drug moiety conjugated to the subject antibody is selected from the group consisting of eribulin or a derivative thereof, such as eribulin mesylate. As used herein, the term "eribulin" refers to a synthetic analog of halichondrin B (a macrocyclic compound originally isolated from the sponge *Halichondria okadais*). Eribulin is a tubulin inhibitor and is believed to bind tubulin and cause cell cycle arrest in the G2/M phase by inhibiting mitotic spindle components. The term "eribulin mesylate" refers to the mesylate salt of eribulin, which is sold under the trade name Halaven^{™}.

In the third aspect of the present disclosure, the present disclosure provides a method for preparing the compound, a pharmaceutically acceptable salt, a stereoisomer or a solvate thereof as described in the first aspect.

In some embodiments, the method comprises: performing an amidation reaction of L¹-L²-OH and eribulin to obtain the compound represented by Formula (I), a pharmaceutically acceptable salt thereof, a stereoisomer thereof, or a solvate thereof, wherein L¹ and L² are as defined in the first aspect of the present disclosure.

In some specific embodiments, the method comprises:
Step 1: dissolving a maleimide active ester with 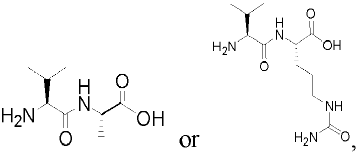 and an alkali in NMP, stirring at room temperature for 4 hours, then HPLC showing the formation of a new peak, and performing Pre-HPLC purification to obtain the target product that is confirmed to be the target product by LCMS.
Step 2: adding 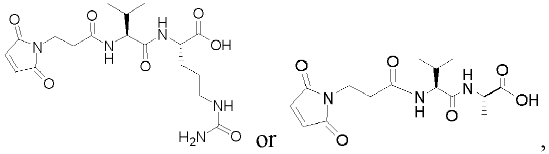 eribulin, HATU, and an alkali to NMP, stirring at room temperature for 2 hours, then HPLC showing the formation of a new peak, and performing Pre-HPLC purification and lyophilization to obtain the target product that is confirmed to be the target product by LCMS.

In the fourth aspect of the present disclosure, the present disclosure provides a method for preparing the antibody-drug conjugate, a pharmaceutically acceptable salt, a stereoisomer or a solvate thereof as described in the second aspect.

In some embodiments, the method comprises: reacting the compound, a pharmaceutically acceptable salt thereof, a stereoisomer thereof, or a solvate thereof as described in the first aspect with the aforementioned Ab to obtain the antibody-drug conjugate represented by Formula (II), a pharmaceutically acceptable salt thereof, a stereoisomer thereof, or a solvate thereof.

In some embodiments, the compound, a pharmaceutically acceptable salt thereof, a stereoisomer thereof, or a solvate thereof as described in the first aspect is prepared by the method as described in the third aspect.

In some embodiments, the produced antibody-drug conjugate (II) may be subjected to operations such as concentration, buffer exchange, purification by using the following Common Operations.

### Common Operation A: Concentration of antibody or antibody-drug conjugate aqueous solution

The antibody or antibody-drug conjugate solution was placed into a container, and subjected to centrifugation (e.g., centrifugation at 2000G to 3800G for 5 to 20 minutes) by using a centrifuge to concentrate the antibody or antibody-drug conjugate solution.

### Common Operation B: Determination of antibody concentration

A UV meter is used to determine the antibody concentration according to the manufacturer's method.

At this time, different 280nm absorption coefficients (1.3mLmg⁻¹cm⁻¹ to 1.8mLmg⁻¹cm⁻¹) are used for different antibodies.

### Common Operation C-1: Buffer exchange of antibody

Following the manufacturer's instructions, phosphate buffer (e.g., PBS) containing sodium chloride (e.g., 137mM) and ethylenediaminetetraacetic acid (EDTA) (e.g., 5mM) (also referred to in the present disclosure as PBS/EDTA) is used to equilibrate PD-10 columns with Sephadex G-25 support. Each of the PD-10 columns is loaded with 1 mL of antibody aqueous solution, and then the fraction (3.5 mL) eluted with 2 mL of PBS/EDTA is separated. This fraction is concentrated using Common Operation A, the antibody concentration is determined using Common Operation B, and then the antibody concentration is adjusted to 10 mg/mL using PBS/EDTA.

### Common Operation C-2: Buffer exchange of antibody

According to the method specified by the manufacturer, phosphate buffer (e.g., 50mM, pH6.5, also called PBS6.5/EDTA in the present disclosure) containing sodium chloride (e.g., 50mM) and EDTA (e.g., 2mM) is used to equilibrate PD-10 columns with Sephadex G-25 support. Each of the PD-10 columns is loaded with 1 mL of antibody aqueous solution, and then the fraction (3.5 mL) eluted with 2 mL of PBS6.5/EDTA is separated. The fraction is concentrated using Common Operation A, the antibody concentration is determined using Common Operation B, and then the antibody concentration is adjusted to 5 mg/mL using PBS6.5/EDTA.

### Common Operation D-1: Purification of antibody-drug conjugate

PD-10 column is equilibrated using any one of buffers selected from the group consisting of commercially available phosphate buffer (e.g., PBS7.4), sodium phosphate buffer (e.g., 10mM, pH6.5; also referred to as PBS6.5 in the present disclosure) containing sodium chloride (e.g., 137mM), or acetate buffer (e.g., 10mM, pH 5.5; also referred to as ABS in the present disclosure) containing sorbitol (e.g., 5%), or MES 25mM pH 6.5, or His 10mM pH 5.5. The column is filled with an antibody-drug conjugate reaction aqueous solution (e.g., about 1 mL), and eluted with an amount of buffer specified by the manufacturer to separate and obtain an antibody fraction, thereby obtaining an antibody-drug conjugate from which unlinked drug linker, low molecular compounds (e.g., tris(2-carboxyethyl)phosphine hydrochloride (TCEP), cysteine, dimethyl sulfoxide, etc.) are removed.

### Common Operation D-2: Purification of antibody-drug conjugate

AKTA column (packing: sephadex G 25) is equilibrated using any one of buffers selected from the group consisting of phosphate buffer (e.g., PBS7.4), sodium phosphate buffer (e.g., 10mM, pH6.5; also referred to as PBS6.5 in the present disclosure) containing sodium chloride (e.g., 137mM), or acetate buffer (e.g., 10mM, pH 5.5; also referred to as ABS in the present disclosure) containing sorbitol (e.g., 5%), or MES 25mM pH 6.5, or His 10mM pH 5.5. An antibody-drug conjugate reaction aqueous solution (e.g., about 2 mL) is loaded by using a sample injector, and eluted with an amount of buffer specified by the manufacturer to separate and obtain an antibody fraction. Thus, an antibody-drug conjugate is obtained from which unlinked drug linker, low molecular compounds (e.g., tris(2-carboxyethyl)phosphine hydrochloride (TCEP), cysteine, dimethyl sulfoxide, etc.) are removed.

In the fifth aspect of the present disclosure, the present disclosure provides a pharmaceutical composition, which comprises the compound, a pharmaceutically acceptable salt, a stereoisomer, or a solvate thereof as described in the first aspect, or the antibody-drug conjugate, a pharmaceutically acceptable salt, a stereoisomer, or a solvate thereof as described in the second aspect, and one or more pharmaceutical adjuvants, such as carriers and/or excipients.

The pharmaceutical composition can be formulated into any pharmaceutically acceptable dosage form. The pharmaceutical composition may also be administered to an individual in need of such treatment in any suitable mode of administration, such as oral, parenteral, rectal or pulmonary administration. When used for oral administration, the pharmaceutical composition can be made into conventional solid preparations, such as tablets, capsules, pills, granules, etc.; or can also be made into oral liquid preparations, such as oral solutions, oral suspensions, syrup, etc. When making oral preparations, appropriate fillers, binders, disintegrants, lubricants, etc. can be added. When used for parenteral administration, the pharmaceutical composition can be made into injections, including injection liquid, sterile powder for injection and concentrated solution for injection. When making injections, conventional methods in the existing pharmaceutical field can be used. When preparing injections, no additives may be added, or appropriate additives may be added according to the properties of drugs. When used for rectal administration, the pharmaceutical composition can be made into suppositories and the like. When used for pulmonary administration, the pharmaceutical composition can be made into inhalants or sprays.

In the sixth aspect of the present disclosure, the present disclosure provides use of the compound, a pharmaceutically acceptable salt, a stereoisomer, or a solvate thereof as described in the first aspect, or the antibody-drug conjugate, a pharmaceutically acceptable salt, a stereoisomer, or a solvate thereof as described in the second aspect, or the pharmaceutical composition as described in the fifth aspect in the manufacture of a medicament for treating and/or preventing of a disease (e.g., cancer disease) associated with abnormal cell activity.

In the seventh aspect of the present disclosure, the present disclosure provides the compound, a pharmaceutically acceptable salt, a stereoisomer, or a solvate thereof as described in the first aspect, or the antibody-drug conjugate, a pharmaceutically acceptable salt, a stereoisomer, or a solvate thereof as described in the second aspect, or the pharmaceutical composition as described in the fifth aspect, for use in treating and/or preventing of a disease (e.g., cancer disease) associated with abnormal cell activity.

In the eighth aspect of the present disclosure, the present disclosure provides a method of treating or preventing a disease (e.g., a cancer disease) associated with abnormal cell activity, comprising administering to an individual in need thereof an effective dose of the compound, a pharmaceutically acceptable salt, a stereoisomer, or a solvate thereof as described in the first aspect, or the antibody-drug conjugate, a pharmaceutically acceptable salt, a stereoisomer, or a solvate thereof as described in the second aspect, or the pharmaceutical composition as described in the fifth aspect. Dosage regimens can be adjusted to provide the best desired response. For example, a single bolus may be administered, several divided doses may be administered over time, or the dosage may be proportionally reduced or increased as the exigencies of therapeutic situation indicate. Dosage values may vary depending on the type and severity of the condition to be alleviated, and may include single or multiple doses. It is further understood that for any particular individual, specific dosage regimens may be adjusted over time according to the individual needs and the professional judgment of the person administering the composition or supervising the administration of the composition.

Various embodiments may involve the use of the subject method and composition to treat cancer, including in-situ cancer or metastatic cancer. Therefore, with regard to the above-mentioned sixth aspect, seventh aspect, and eighth aspect, in some embodiments, the cancer in-situ cancer or metastatic cancer. In some specific embodiments, the cancer includes, but is not limited to, metastatic breast cancer, non-small cell lung cancer, Burkitt lymphoma, Hodgkin's lymphoma, acute myeloid leukemia, chronic myeloid leukemia, acute lymphoblastic leukemia, chronic lymphoblastic leukemia, skin cancer, oral cancer, esophageal cancer, gastrointestinal cancer, lung tract cancer, lung cancer, gastric cancer, colon cancer, rectal cancer, triple negative breast cancer (TNBC), ovarian cancer, prostate cancer, uterine cancer, endometrial cancer, cervical cancer, bladder cancer, pancreatic cancer, bone cancer, brain cancer, connective tissue cancer, thyroid cancer, liver cancer, gallbladder cancer, bladder (urothelial) cancer, kidney cancer, skin cancer, central nervous system cancer, testicular cancer, epithelial cancer, head and neck cancer. In some preferred embodiments, the cancer is selected from the group consisting of metastatic breast cancer, TNBC, non-TNBC, cervical cancer, endometrial cancer, lung cancer, ovarian cancer, bladder (urothelial) cancer, colon cancer, and pancreatic cancer. In some more preferred embodiments, the cancer is TNBC, ovarian cancer, cervical cancer, bladder (urothelial) cancer, or pancreatic cancer (e.g., in-situ pancreatic cancer).

In certain embodiments involving the treatment of cancer, the antibody or immunoconjugate can be used in combination with surgery, radiation therapy, chemotherapy, immunotherapy performed using naked antibody including checkpoint inhibitory antibody, radioimmunotherapy, immunomodulator, vaccine, etc. Most preferably, the antibody or immunoconjugate is used in combination with PARP inhibitor, tubulin inhibitor, Bruton's kinase inhibitor and/or PI3K inhibitor. These combination therapies may allow lower doses of therapeutic agents to be administered in the context of the combination, thereby reducing some severe side effects and potentially reducing the duration of treatment required. The respective full dose may also be administered when there is no or minimal overlapping toxicity.

Although the antibody or immunoconjugate may be administered as a periodic bolus injection, in alternative embodiments, the antibody or immunoconjugate may be administered by continuous infusion. To increase the Cmax of the antibody or immunoconjugate in the blood and prolong the PK of the antibody or immunoconjugate in the blood, continuous infusion can be administered, for example, through an indwelling catheter. Such devices are known in the art, such as HICKMAN^{®}, BROVIAC^{®} or Port-A-Cath^{®} catheters (see, for example, Skolnik et al., Ther DrugMonit 32:741-48, 2010), and any of the known indwelling catheters can be used. A variety of continuous infusion pumps are also known in the art, and any such known infusion pump may be used. The dose range for continuous infusion may be between 0.1 and 3.0 mg/kg per day. More preferably, these immunoconjugates are administered by intravenous infusion over a relatively brief period of 2 to 5 hours, more preferably 2 to 3 hours.

In particularly preferred embodiments, the antibody or immunoconjugate and dosing schedule may be effective in patients who are resistant to standard therapies. For example, an hRS7-eribulin immunoconjugate can be administered to a patient who has not responded to prior therapy with eribulin. Strikingly, irinotecan-resistant patients may show partial or even complete responses to hRS7-eribulin. The ability of immunoconjugate to specifically target tumor tissue may overcome tumor resistance due to improved targeting and enhanced delivery of therapeutic agent. A particularly preferred subject may be one who has Trop-2 positive breast cancer, ovarian cancer, cervical cancer, endometrial cancer, lung cancer, prostate cancer, colon cancer, rectal cancer, gastric cancer, esophageal cancer, bladder (urothelial) cancer, kidney cancer, pancreatic cancer, brain cancer, thyroid cancer, epithelial cancer, or head and neck cancer. Preferably, the cancer is metastatic cancer. More preferably, the patient has previously failed treatment with at least one standard anti-cancer therapy. In alternative preferred embodiments, the cancer is metastatic breast cancer, TNBC, non-TNBC, endometrial cancer, lung cancer, ovarian cancer, or colon cancer.

In the ninth aspect of the present disclosure, the present disclosure provides a pharmaceutical preparation, which comprises the compound, a pharmaceutically acceptable salt, a stereoisomer, or a solvate thereof as described in the first aspect, or the conjugate, a pharmaceutically acceptable salt, a stereoisomer, or a solvate thereof as described in the second aspect, or the pharmaceutical composition as described in the fifth aspect.

In the tenth aspect of the present disclosure, the present disclosure provides use of the compound, a pharmaceutically acceptable salt, a stereoisomer, or a solvate thereof as described in the first aspect, or the conjugate, a pharmaceutically acceptable salt, a stereoisomer, or a solvate thereof as described in the second aspect, or the pharmaceutical composition as described in the fifth aspect in the manufacture of a pharmaceutical preparation.

In the eleventh aspect of the present disclosure, the present disclosure provides a kit, which comprises the compound, a pharmaceutically acceptable salt, a stereoisomer, or a solvate thereof as described in the first aspect, or the conjugate, a pharmaceutically acceptable salt, a stereoisomer, or a solvate thereof as described in the second aspect, or the pharmaceutical composition as described in the fifth aspect, or the pharmaceutical preparation as described in the ninth aspect.

The aforementioned compound, antibody-drug conjugate, pharmaceutically acceptable salt, stereoisomer, or solvate, pharmaceutical preparation or kit provided by the present disclosure can be used to inhibit the growth, proliferation or migration of cancer cells. The compound, antibody-drug conjugate, pharmaceutically acceptable salt, stereoisomer, or solvate, pharmaceutical preparation or kit is used for in vivo or in vitro administration; for example, it is administered to a subject, or is administered to cells in vitro (e.g., cell lines, or cells from an individual, such as cancer cells). In some preferred embodiments, the aforementioned compound, antibody-drug conjugate, pharmaceutically acceptable salt, stereoisomer, or solvate, pharmaceutical preparation or kit is used for non-therapeutic purposes (e.g., in vitro scientific research, etc.), used for inhibiting the growth, proliferation or migration of cancer cells.

Thus, in the twelfth aspect of the present disclosure, the present disclosure provides use of the compound, a pharmaceutically acceptable salt, a stereoisomer, or a solvate thereof as described in the first aspect, or the antibody-drug conjugate, a pharmaceutically acceptable salt, a stereoisomer, or a solvate thereof as described in the second aspect, or the pharmaceutical composition as described in the fifth aspect, or the pharmaceutical preparation as described in the ninth aspect in the manufacture of a reagent for inhibiting the growth, proliferation or migration of a cancer cell, especially in the manufacture of a reagent for inhibiting the growth, proliferation or migration of a cancer cell in vitro. Wherein, the carcinoma in the cancer cell is cancer, and in some embodiments, the cancer is as described above. In some embodiments, the use is for non-therapeutic purposes.

In the thirteenth aspect of the present disclosure, the present disclosure provides the compound, a pharmaceutically acceptable salt, a stereoisomer, or a solvate thereof as described in the first aspect, or the antibody-drug conjugate, a pharmaceutically acceptable salt, a stereoisomer, or a solvate thereof as described in the second aspect, or the pharmaceutical composition as described in the fifth aspect, or the pharmaceutical preparation as described in the ninth aspect, for use in inhibiting the growth, proliferation or migration of a cancer cell, especially for use in inhibiting the growth, proliferation or migration of a cancer cell in vitro. Wherein, the carcinoma in the cancer cell is cancer, and in some embodiments, the cancer is as described above. In some embodiments, the use is for non-therapeutic purposes.

In the fourteenth aspect of the present disclosure, the present disclosure provides a method for inhibiting the growth, proliferation or migration of a cancer cell (especially for inhibiting the growth, proliferation or migration of a cancer cell in vitro), which comprises administering to the cancer cell an effective amount of the compound, a pharmaceutically acceptable salt, a stereoisomer, or a solvate thereof as described in the first aspect, or the antibody-drug conjugate, a pharmaceutically acceptable salt, a stereoisomer, or a solvate thereof as described in the second aspect, or the pharmaceutical composition as described in the fifth aspect, or the pharmaceutical preparation as described in the ninth aspect. Wherein, the carcinoma in the cancer cell is cancer, and in some embodiments, the cancer is as described above. In some embodiments, the method is used for non-therapeutic purposes.

Specifically, with regard to the above-mentioned twelfth aspect, thirteenth aspect, and fourteenth aspect, in some embodiments, the cancer cell is an in-situ cancer cell or metastatic cancer cell. In some specific embodiments, the cancer cell a cancer cell of the cancer selected from the group consisting of metastatic breast cancer, non-small cell lung cancer, Burkitt lymphoma, Hodgkin's lymphoma, acute myeloid leukemia, chronic myeloid leukemia, acute lymphoblastic leukemia, chronic lymphoblastic leukemia, skin cancer, oral cancer, esophageal cancer, gastrointestinal cancer, lung tract cancer, lung cancer, gastric cancer, colon cancer, rectal cancer, triple negative breast cancer (TNBC), ovarian cancer, prostate cancer, uterine cancer, endometrial cancer, cervical cancer, bladder cancer, pancreatic cancer, bone cancer, brain cancer, connective tissue cancer, thyroid cancer, liver cancer, gallbladder cancer, bladder (urothelial) cancer, kidney cancer, skin cancer, central nervous system cancer, testicular cancer, epithelial cancer, and head and neck cancer. In some preferred embodiments, the cancer cells are cancer cells of the cancer selected from the group consisting of metastatic breast cancer, TNBC, non-TNBC, cervical cancer, endometrial cancer, lung cancer, ovarian cancer, bladder (urothelial) cancer, colon cancer, and pancreatic cancer. In some more preferred embodiments, the cancer cell is a cancer cell of the cancer selected from the group consisting of TNBC, ovarian cancer, cervical cancer, bladder (urothelial) cancer, and pancreatic cancer (e.g., in-situ pancreatic cancer cell).

Unless otherwise defined, all technical and scientific terms used herein are intended to have the same meaning as commonly understood by one of ordinary skill in the art. References to technology as used herein are intended to mean technology as commonly understood in the art, including those variations or equivalent technology that would be apparent to those skilled in the art. Although the following terms are believed to be well understood by those skilled in the art, the following definitions are set forth to better explain the present invention.

In the present invention, Y is 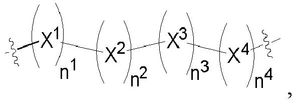 n¹, n², n³ and n⁴ are each independently selected from the group consisting of 0 and 1, and at least one of n¹, n², n³ and n⁴ is 1. Wherein, for example, when n¹ is 0 and n², n³, and n⁴ are 1, Y is 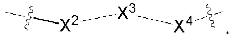 For another example, when n² is 0 and n¹, n³, and n⁴ are 1, Y is 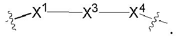 Or for example, when n¹ and n² are 1 and n³ and n⁴ are 0, Y is 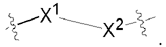 Other similar definitions can be understood with reference to the foregoing contents.

In the present invention, the term "amino acid residue" refers to a remaining incomplete amino acid structure that is formed after the amino group of an amino acid loses a hydrogen and the carboxyl group of the amino acid loses a hydroxyl group, and has an amino end and a carbonyl end. In the present invention, L² is an amino acid residue or a short peptide composed of 2 to 10 (preferably 2 to 4) amino acid residues, wherein the types of 2 to 10 (preferably 2 to 4) amino acids can be the same or different from each other. For example, if L² is a short peptide consisting of 4 amino acid residues, and the amino acids are selected from the group consisting of glycine and phenylalanine, then L² can be the following short peptide or peptide residue: glycine-glycine-phenylalanine-glycine (Gly-Gly-Phe-Gly), specifically, it can be

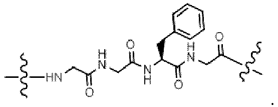

In the present invention, unless otherwise clearly stated, the descriptions "each of ... is independently selected from" and "... are each independently selected from" used throughout the present disclosure are interchangeable and should be understood in a broad sense, and refer to that the specific options expressed by the same or different symbols in different groups do not affect each other, and can also refer to that the specific options expressed by the same or different symbols in the same group do not affect each other.

The "linker", "linker structure" or "connector" or "linker unit" mentioned in the present invention refers to a chemical structural fragment or bond that is linked to an antibody at one end and linked to a drug (drug compound) at the other end, and can also be linked to other linkers before being linked to the drug compound. The linker structure of the present invention can be synthesized by methods known in the art, or can be synthesized using the method described in the present invention.

The "antibody-drug conjugate" of the present invention, that is, ADC, refers to that a targeting moiety such as an antibody or antigen-binding fragment being linked to a biologically active drug through a stable linker unit.

In the present invention, "pharmaceutically acceptable salt" refers to a relatively non-toxic acid addition salt or base addition salt of the compound or conjugate of the present invention. The acid addition salt is a salt formed by the compound or conjugate of the present invention and a suitable inorganic acid or organic acid, and these salts can be prepared by reacting the compound or conjugate of the present invention with a suitable organic acid or inorganic acid in an appropriate solvent. Representative acid addition salts include hydrobromide, hydrochloride, sulfate, bisulfate, sulfite, acetate, oxalate, valerate, oleate, palmitate, stearate, laurate, borate, benzoate, lactate, nitrate, phosphate, hydrogen phosphate, carbonate, bicarbonate, toluate, citrate, maleate, fumarate, succinate, malate, ascorbate, tannate, pamoate, alginate, naphthalenesulfonate, tartrate, benzoate, methanesulfonate, p-toluenesulfonate, gluconate, lactobionate and laurylsulfonate, etc. The base addition salt is a salt formed by the compound or conjugate of the present invention and a suitable inorganic base or organic base, and these salts can be prepared by reacting the compound or conjugate of the present invention with a suitable inorganic base or organic base in an appropriate solvent. Representative base addition salts include, for example, salts formed with alkali metal, alkaline earth metal, quaternary ammonium cation, such as sodium salt, lithium salt, potassium salt, calcium salt, magnesium salt, tetramethyl quaternary ammonium salt, tetraethyl quaternary ammonium salt, etc.; amine salts include salts formed with ammonia (NH₃), primary amine, secondary amine or tertiary amine, such as methylamine salt, dimethylamine salt, trimethylamine salt, triethylamine salt, ethylamine salt, etc.

The compound or conjugate of the present invention may exist in a specific geometric or stereoisomer form. In the compound or conjugate of the present invention, a chiral center may exist in the drug, in the linker structure, or in the antibody or derivative thereof. In the present invention, all such compounds include cis and trans isomers, (-)- and (+)-enantiomers, (R)- and (S)-enantiomers, diastereomer, (D)-isomer, (L)-isomer, as well as racemic mixture and other mixture thereof, such as enantiomer- or diastereomer-enriched mixture, all fall into the scope of the present invention.

The optically active (R)- and (S)-isomers as well as the D and L isomers can be prepared by chiral synthesis or chiral reagents or other conventional techniques. If it is desired to obtain an enantiomer of the compound or conjugate of the present invention, it can be prepared by asymmetric synthesis or derivatization with chiral auxiliary, in which the resulting diastereomeric mixture is separated and the auxiliary group is cleaved to provide the pure enantiomer as desired. Alternatively, when the molecule contains a basic functional group (e.g., an amino group) or an acidic functional group (e.g., a carboxyl group), a diastereomeric salt is formed with a suitable optically active acid or base, and then diastereomeric resolution is performed by conventional methods known in the art, and pure enantiomers can be recovered and obtained. Furthermore, the separation of enantiomers and diastereomers is usually accomplished by chromatography using chiral stationary phases, optionally in combination with chemical derivatization methods (e.g., generation of carbamate from amine).

In the present invention, the solvates (e.g., hydrates) of the compound, conjugate, pharmaceutically acceptable salt, or stereoisomer thereof of the present invention are also within the scope of the present invention. Specific examples of suitable solvates include solvates of the compound or conjugate of the present invention that are formed with acetone, 2-butanol, 2-propanol, ethanol, ethyl acetate, tetrahydrofuran, diethyl ether, and the like. Hydrates and ethanolates may also be listed.

As used herein, "treatment" of an individual suffering from a disease or disease condition means that the individual's symptoms are partially or completely alleviated, or remain unchanged following treatment. Treatment therefore includes prevention, treatment and/or cure. "Prevention" means preventing an underlying disease and/or preventing the worsening of symptoms or progression of a disease. Treatment or prevention also comprises any pharmaceutical use of the ADC, pharmaceutical composition, and pharmaceutical preparation provided herein.

In the present disclosure, the term "effective dose" refers to an amount of a compound that, when administered, alleviates to a certain extent one or more symptoms of the condition being treated. Similarly, the term "effective amount" refers to an amount of a compound that, when administered, inhibits the growth, proliferation, or migration of cancer cells to a certain extent.

As used herein, "therapeutic effect" refers to an effect resulting from treatment of an individual that alters, generally improves or ameliorates the symptoms of a disease or disease condition, or cures a disease or disease condition.

In the present invention, "individual" comprises a human or non-human animal. Exemplary human individual comprises human subject (referred to as patient) suffering from a disease (e.g., the disease described herein), or normal subject. In the present invention, "non-human animal" comprises all vertebrates, such as non-mammals (e.g., birds, amphibians, reptiles) and mammals, such as non-human primates, domestic animals and/or domesticated animals (e.g., sheep, dogs, cats, cows, pigs, etc.).

It is to be understood that the disclosed compositions and methods are not limited to the specific compositions and methods described and/or illustrated herein, and that the terminology used herein is for the purpose of describing particular embodiments by way of example only and is not intended to limit the claimed compositions and methods.

Throughout the present disclosure, compositions and methods of using the compositions are described. Where the present disclosure describes or claims features or embodiments in connection with a composition, such features or embodiments apply equally to methods of using the composition. Likewise, where the present disclosure describes or claims features or embodiments in connection with methods of using a composition, such features or embodiments apply equally to the composition.

When a range of values is stated, it comprises embodiments using any specific value within the stated range. Furthermore, references to values within a range comprise every value within that range. All ranges include their endpoints and can be combined. When values are expressed as approximations, by use of the preceding "about," it is understood that the particular value forms another embodiment. Unless the context clearly dictates otherwise, references to a specific numerical value comprise at least the stated specific value. The use of "or" will mean "and/or" unless the specific context in which it is used dictates otherwise. All references cited herein are incorporated by reference for any purpose. In the event of a conflict between a reference and the present disclosure, the present disclosure will prevail.

It is understood that, for clarity, certain features of the disclosed compositions and methods that are described herein in the context of separate embodiments may also be provided in combination in a single embodiment. Conversely, for simplicity, various features of the disclosed compositions and methods that are described in the context of a single embodiment may also be provided separately or in any subcombination.

Documents mentioned in this text are incorporated herein by reference in their entirety.

### Beneficial effects:

1. The ADC prepared by the linker designed in the present disclosure not only has better efficacy than the ADC containing the PAB structure, but also simplifies the structure of the linker, reduces the synthesis steps, and reduces the cost.
2. In the linker of the present disclosure, the length of the chain linking the maleimide group and the amino acid residue or short peptide is shortened, for example, to 3 methylene groups or even 2 methylene groups, and the hydrophobic group PAB is removed, so that the hydrophilicity of the ADC molecule is improved, the formation of ADC aggregate is reduced, the stability of ADC is improved, and its PK in vivo is improved as well, and the half-life of the drug in vivo is increased.
3. In the present disclosure, it is unexpectedly discovered that the ADC constructed using the truncated linker of the present disclosure can significantly improve the affinity of the antibody to the target protein.
4. The ADC constructed by the truncated linker of the present disclosure has improved efficacy in inhibiting tumors (such as pancreatic cancer or colon cancer).
5. The ADC of the present disclosure (e.g., ADC-1.1) has a bystander effect. For example, the ADC of the present disclosure (e.g., ADC-1.1) can not only kill target cells that highly express TROP-2 in tumor cells, but also can exert a certain degree of killing effect on nearby TROP-2 negative cells.

### Brief Description of the Drawings

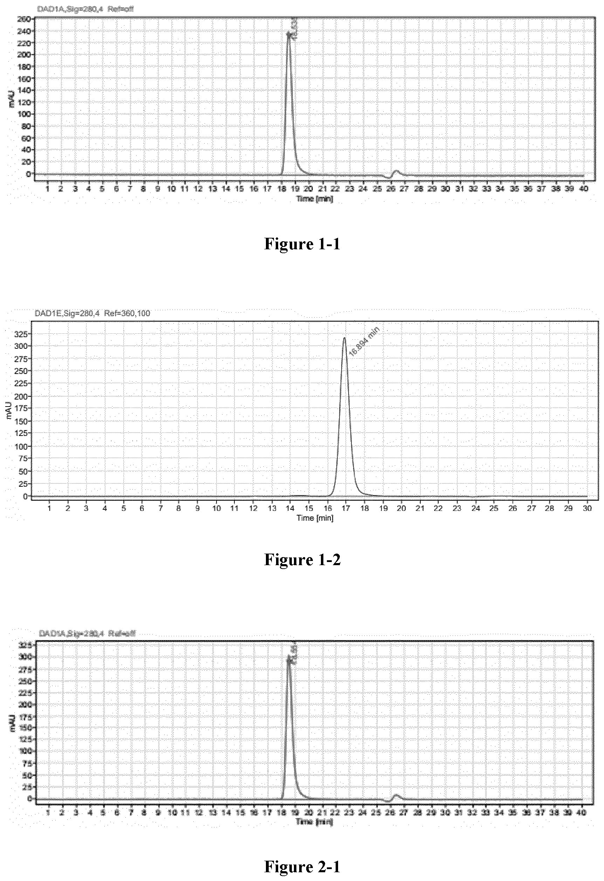

Figure 1-1 shows the SEC-HPLC detection chart of ADC-1.1 according to the example of the present invention.
Figure 1-2 shows the SEC-HPLC detection chart of ADC-1.2 according to the example of the present invention.
Figure 2-1 shows the SEC-HPLC detection chart of ADC-2.1 according to the example of the present invention.
Figure 2-2 shows the SEC-HPLC detection chart of ADC-2.2 according to the example of the present invention.
Figure 3 shows the SEC-HPLC detection chart of Control Conjugate 2 (i.e. ADC-5) of the present invention.
Figure 4 shows the SEC-HPLC purity detection chart of Control Conjugate ADC-217 of the present invention.
Figure 5 shows the result chart of using a protein hydrophobic interaction chromatography column (HIC) to detect the hydrophilicity and hydrophobicity of various ADCs of the present invention.
Figure 6 shows the result chart of detection of affinity of various ADCs of the present invention to TROP protein.
Figure 7 shows the statistical curve chart of the anti-tumor activity of various ADCs of the present invention.
Figure 8 shows the body weight change curve chart of experimental animals after treatment with various ADCs of the present invention.
Figure 9 shows the tumor photographs taken from experimental animals after treatment with various ADCs of the present invention.
Figure 10 shows the bystander effect result chart of the ADC-1.1 of the present invention.
Figure 11 shows the statistical curve chart of anti-tumor activity of various ADCs of the present invention.
Figure 12 shows the body weight change curve chart of experimental animals after treatment with various ADCs of the present invention.
Figure 13 shows the tumor photographs of experimental animals after treatment with various ADCs of the present invention.

### Specific Models for Carrying Out the present invention

The embodiments of the present invention will be described in detail below with reference to the accompanying drawings and examples. However, those skilled in the art will understand that the following drawings and examples are only used to illustrate the present invention and do not limit the scope of the present invention. Various objects and advantageous aspects of the present invention will become apparent to those skilled in the art from the accompanying drawings and the following detailed description of preferred embodiments. In the following examples, unless otherwise specified, various reagents were commercially available.

### Example 1: Preparation and detection of hRS7 antibody

### 1. Gene synthesis, transfection and antibody preparation

hRS7 antibody was produced in CHO cells. Expression vectors containing hRS7 antibody genes were constructed using conventional molecular biology methods. The amino acid sequences of the hRS7 antibody light chain and heavy chain were shown in SEQ ID NO: 1 and SEQ ID NO: 2, respectively, and their corresponding nucleotide sequences were shown in SEQ ID NO:3 and SEQ ID NO:4, respectively. The above two sequences were inserted into the same expression vector, a large amount of transfection plasmid was extracted and prepared. It was then transfected into CHO-K1 cells (ATCC CCL-61). The specific transfection and antibody preparation processes were as follows:
(1) Cell culture: CHO-K1 cells were grown in suspension in ActiPro (GE HyClone) medium and cultured at 37°C, 7% CO₂, 140 rpm, and 90% relative humidity;
(2) Transfection: After entering the logarithmic growth phase, the cells were taken and centrifuged, and resuspended in fresh ActiPro culture medium, counted and adjusted to reach a cell density of 1.2×10⁷ cells/ml. 500 µl of the cell suspension was transferred into an electroporation cup, then added with 40 µg of the constructed plasmid, the cells and plasmid were mixed well, and then the plasmid was introduced by electroporation (Bio-rad electroporation instrument);
(3) Subcloning: The cells after electroporation were resuspended in ActiPro medium at 37°C, and distributed at 100 µl per well into a 96-well plate. The cell supernatant was assayed to determine antibody expression level. The clones with higher expression levels were transferred from the 96-well plate to a 24-well plate for culture, and then transferred to a 6-well plate for culture. The antibody production and productivity of the cells were measured. The four clones with the highest expression levels were selected for subcloning, and then transferred to a shake flask, and continuously cultured in an incubator.

### 2 Antibody purification

The fluid of high expression cells cultured in the shake flask was collected, and subjected to protein A affinity purification (GE, Mab Select SuRe) and ion exchange purification (GE, Capto S). The purified antibodies were analyzed using SDS-PAGE and SEC-HPLC for molecular weight and purity. The SDS-PAGE measurement results showed that the molecular weight of the prepared hRS7 was in line with expectation, and the antibody purity measured by SEC-HPLC was 99.1%.
Amino acid sequence of hRS7 antibody light chain 
DIQLTQSPSSLSASVGDRVSITCKASQDVSIAVAWYQQKPGKAPKLLIYSASYRYTG
VPDRFSGSGSGTDFTLTISSLQPEDFAVYYCQQHYITPLTFGAGTKVEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC (SEQ ID NO:1)
Amino acid sequence of hRS7 antibody heavy chain 
QVQLQQSGSELKKPGASVKVSCKASGYTFTNYGMNWVKQAPGQGLKWMGWINT
YTGEPTYTDDFKGRFAFSLDTSVSTAYLQISSLKADDTAVYFCARGGFGSSYWYFDVW
GQGSLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKRVEPKSCDKTHTC
PPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 2)
Nucleotide sequence of hRS7 antibody light chain 
gacatccagc tgacccagtc cccatcttct ctgtccgcta gtgtgggcga cagagtgtct

atcacatgca aggcgtctca ggacgtgagc atcgccgtgg cttggtatca gcagaagccc

ggcaaggccc caaagctgct catctactcc gcttcttaca gatacaccgg cgtgcccgac

cggttcagcg gctctggctc tggcacagac ttcacactga caatctctag cttgcagcct

gaggacttcg ccgtgtacta ctgccagcag cactacatca ccccactcac attcggcgct

ggcaccaagg tggagatcaa gagaacagtg gccgctccta gcgtgttcat cttccctcct

agcgacgagc agctgaagag cggcaccgct tccgtggtgt gcctgctgaa caacttctac

cctcgggagg ctaaggtgca gtggaaggtg gacaacgccc ttcaatccgg caactctcag

gagtctgtga ccgagcagga ctctaaggac tccacatact ccctgtcctc cacactgaca

ctgtctaagg ccgactacga gaagcacaag gtgtacgctt gcgaggtgac ccaccagggc

ctcagctctc ctgtgacaaa gtctttcaac cggggcgagt gc (SEQ ID NO: 3)
Nucleotide sequence of hRS7 antibody heavy chain

caggtgcagc tccagcagtc cggcagcgag ctgaagaagc ctggcgcttc tgtgaaggtg

tcttgcaagg cgagtggcta cacattcacc aactacggca tgaactgggt gaagcaggct

cctggccagg gcctcaagtg gatgggctgg atcaacacat acacaggcga gccaacatac

acagacgact tcaagggccg gttcgctttc agcctcgaca cctctgtgtc taccgcttac

ctacagatca gctccctgaa ggccgacgac accgctgtgt acttctgcgc tagaggcggc

ttcggcagct cttactggta cttcgacgtg tggggccagg gctctctcgt gacagtgtct

agcgccagca caaagggccc atccgtgttc ccactcgctc cttcttctaa gtctacatct

ggcggcaccg ccgccctcgg ctgcctcgtg aaggactact tcccagagcc tgtgacagtg
tcttggaact ccggcgccct cacatccggc gtgcacacat tccccgccgt gttacaatct
agcggcctgt actctttaag ctctgtggtg accgtgccat ctagctctct cggcacccag
acctacatct gcaacgtgaa ccacaagcca tctaacacca aggtggacaa gagagtggag
ccaaagtctt gcgacaagac ccacacatgc ccaccttgcc ccgcccccga gctgctgggc
ggcccatctg tgttcctgtt cccacctaag ccaaaggaca cactcatgat cagcagaacc
cccgaggtga catgcgtggt ggtggacgtg tctcacgagg accccgaggt gaagttcaac
tggtacgtgg acggcgtgga ggtgcacaac gctaagacca agccacggga ggagcagtac
aacagcacct accgggtggt gagcgtgctg accgtgctgc accaggactg gctgaacggc
aaggagtaca agtgcaaggt gtccaacaag gctctgcccg cccctatcga gaagacaatc
agcaaggcta agggccagcc tagagagcct caggtgtaca cactgcctcc atctcgggag
gagatgacaa agaaccaggt gagcctcaca tgcctcgtga agggcttcta ccctagcgac
atcgccgtgg agtgggagtc taacggccag cctgagaaca actacaagac cacccctcca
gtgctcgact ccgacggctc tttcttcctg tactctaagc tgacagtgga caagtccaga
tggcagcagg gcaacgtgtt ctcttgctcc gtgatgcacg aggccctcca caaccactac
acccagaagt ccctgtccct gtctcctggc aag (SEQ ID NO: 4)

### Example 2: Preparation of conjugates

### 1-1. Preparation of conjugate ADC-1.1

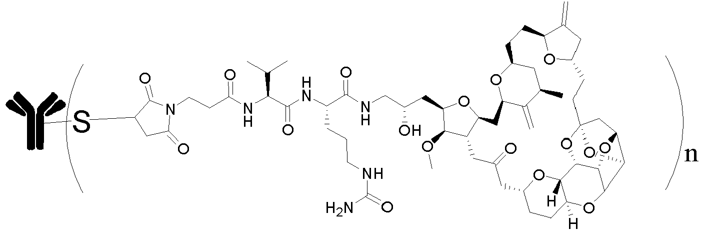

### 1.1.1 Synthesis of Intermediate A

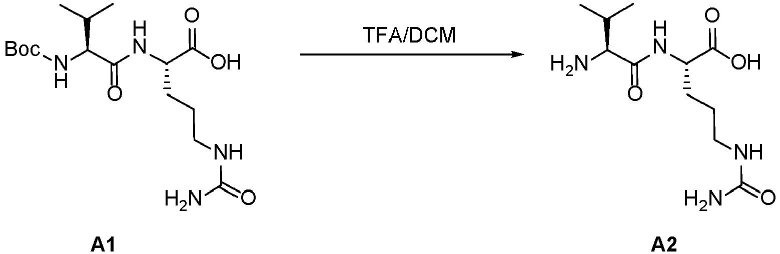

1.1.1.1 Trifluoroacetic acid (1 mL) was added to a solution of A1 (60 mg, 0.16 mmol) in DCM (2 mL). The reaction solution was reacted at room temperature for 3 hours. After spin-drying the reaction solution to dryness and removing the solvent, about 42 mg of the crude target product A2 was obtained with a yield of 98.8%.

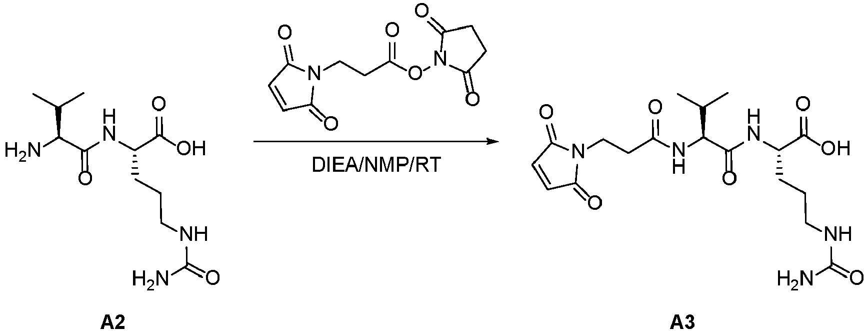

1.1.1.2 To a solution of A2 (42 mg, 0.15 mmol) in NMP (2 mL), TEA (31 mg, 0.31 mmol) and MC(3)-NHS (i.e., 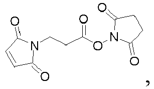 21.14 mg, 0.18 mmol) were added. The reaction solution was reacted at room temperature for 16 hours. The reaction solution was directly purified by Pre-HPLC to obtain about 21 mg of the target product A3, with a yield of 32.24%. LCMS: [M+1]+=426.4.

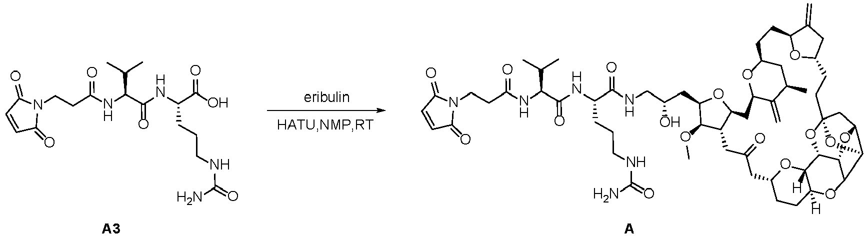

1.1.1.3 TEA (4.76 mg, 0.047 mmol) and HATU (13.41 mg, 0.035 mmol) were added to the solution of A3 (10 mg, 0.023 mmol) in NMP (2 mL). The reaction solution was reacted at room temperature for half an hour, then compound eribulin (8.6 mg, 0.012 mmol) was added, and reacted under stirring at room temperature for 3 hours. The reaction solution was directly purified by Pre-HPLC to obtain about 3 mg of target product A, with a yield of 14.03%. LCMS: [M+1]+=1138.8.

### 1.1.2 Synthesis of crude coupling product ADC-1.1

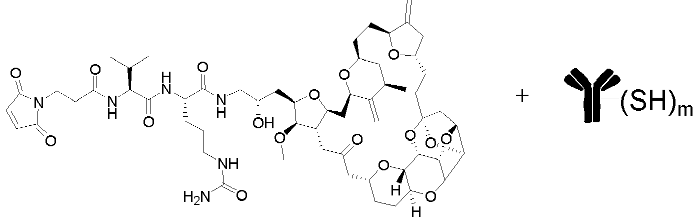

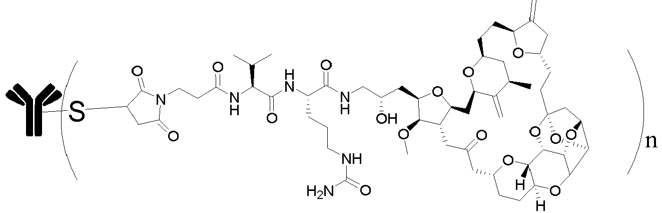

The antibody prepared in Example 1 was added to a pH 7.4 PBS solution at 10 mg/mL, stirred and mixed using a 25°C water bath, and simultaneously added with an equal volume of TCEP solution in 2.4 times molar amount, and the solution was placed in a 25°C water bath and reacted for 1.5 hours. A 25°C water bath was used for the sample, then the antibody solution was stirred and mixed, and simultaneously added with Compound A in 8 times molar amount in DMSO solution (the final concentration of DMSO was 10%), and the reaction was carried out under shaking with a shaker at 25°C for 90 minutes; finally, the sample was stirred and mixed, and simultaneously added with cysteine in 8 times molar amount, and allowed to stand and react in a 25°C water bath for 10 minutes to obtain the coupling product ADC-1.1.

### 1.1.3 Detection of crude coupling product ADC-1.1

The crude product of the coupling reaction was detected by SEC.

SEC chromatography conditions were as follows:
Column model: TSKgel G4000SWxl 7.8mmI.D.*30cm, 8µm
Detector wavelength: 280 nm/220 nm
Column temperature: 30°C
Flow rate: 1 mL/min
Elution mode: isocratic elution
Injection volume: 10 µL
Running time: 55 minutes

### 1.1.4 Purification of coupling reaction product:

After desalting and purification through PD-10 desalting column (packing: sephadex G 25), the conjugate was obtained. The conjugate was then exchanged into a solution of MES 25mM pH5.5 with 5% trehalose. SEC detection showed that small molecules had been completely removed. The detection result of purified ADC-1.1 was shown in Figure 1-1.

### 1.1.5 Determination of DAR

An equal volume of 50mM DTT solution was added to ADC-1.1 and monoclonal antibody solutions, vortexed and mixed well in a 37°C water bath for 30 minutes. The reduced ADC and antibody were analyzed by RP-HPLC for conjugated and unconjugated antibody light and heavy chains. The DAR value was determined to be 3.9 by analyzing their compositions.

### 1-2 Preparation of conjugate ADC-1.2

### 1.2.1 Synthesis of crude coupling product ADC-1.2

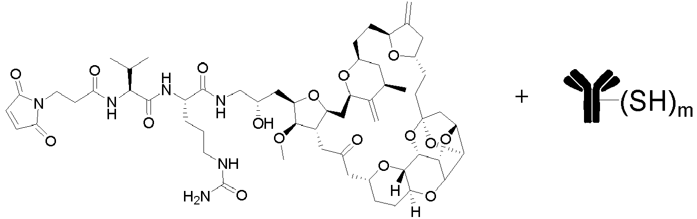

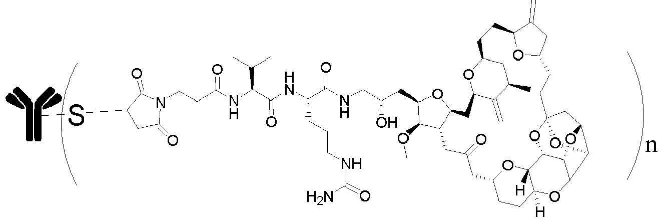

The antibody prepared in Example 1 was added to a pH 7.3 PBS solution at 10 mg/mL, stirred and mixed well using a 22°C water bath, and simultaneously added with an equal volume of TCEP solution in 2.15 times molar amount; the solution was allowed to stand and react for 2 hours in a 22°C water bath. A 22°C water bath was used for the sample, then the antibody solution was stirred and mixed, and simultaneously added with Compound A in 5.5 times molar amount in DMSO solution (the final concentration of DMSO was 10%), the reaction was carried out by shaking in a shaker at 22°C for 60 minutes; finally, the sample was stirred and mixed, and simultaneously added with cysteine in 8 times molar amount, and allowed to stand and react in a 22°C water bath for 10 minutes to obtain the coupling product ADC-1.2.

### 1.2.2 Detection of crude coupling product ADC-1.2

The crude product of the coupling reaction was detected by SEC.

SEC chromatography conditions were as follows:
Column model: TSKgel G4000SWxl 7.8mmI.D.*30cm, 8µm
Detector wavelength: 280 nm/220 nm
Column temperature: 30°C
Flow rate: 1 mL/min
Elution mode: isocratic elution
Injection volume: 10 µL
Running time: 55 minutes

### 1.2.3 Purification of coupling reaction product:

After desalting and purification through PD-10 desalting column (packing: sephadex G 25), the conjugate was obtained. The conjugate was then exchanged into a solution of His 20mM pH 6.0 with 5% sucrose. SEC detection showed that the small molecules had been completely removed. The detection result of purified ADC-1.2 was shown in Figure 1-2.

### 1.2.4 Determination of DAR

An equal volume of 50mM DTT solution was added to the ADC-1.2 and monoclonal antibody solutions, vortexed and mixed well in a 37°C water bath for 30 minutes. The reduced ADC and antibody were analyzed by RP-HPLC for conjugated and unconjugated antibody light and heavy chains. The DAR value was determined to be 3.5 by analyzing their compositions.

### 2-1. Preparation of conjugate ADC-2.1

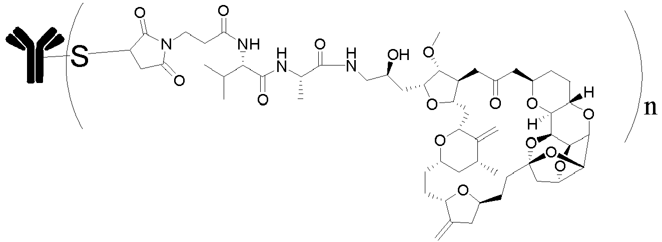

### 2.1.1 Preparation of Intermediate B

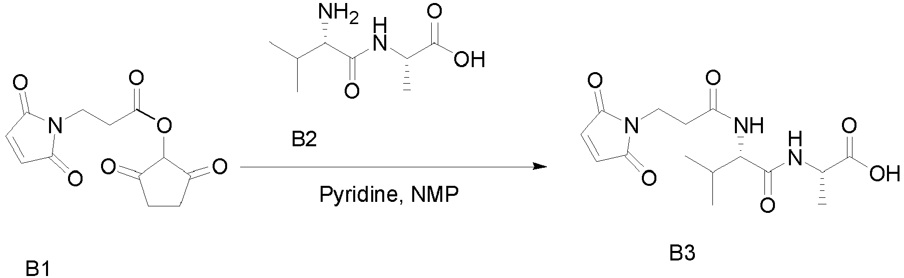

2.1.1.1 B1 (50 mg), B2 (35 mg) and pyridine (40 µL) were dissolved in NMP (1.0 mL), the reaction solution was stirred at room temperature overnight, then purified by Pre-HPLC and lyophilized to obtain about 10 mg of the target compound B3. LCMS: [M+1]+ = 338.4

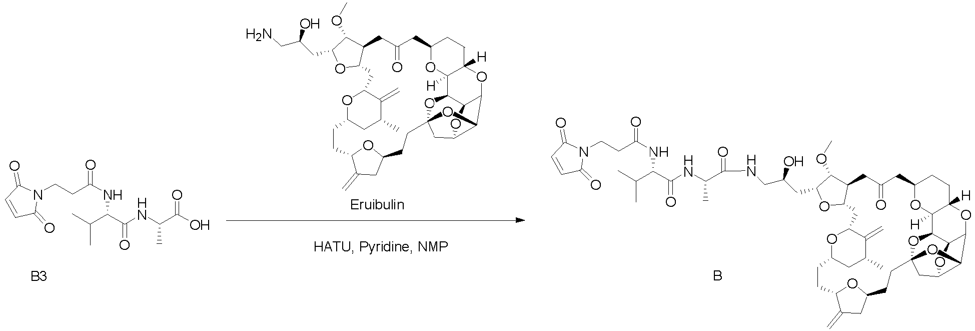

### 2.1.1.2 Synthesis of Mal-Val-Ala-eribulin

B3 (10 mg), eribulin (12 mg), HATU (15 mg) and pyridine (20 µL) were dissolved in NMP (0.5 mL), and stirred at room temperature overnight; then purified by Pre-HPLC to obtain 8 mg of the target product B. LCMS: [M+1]+ = 1051.9

### 2.1.2 Synthesis of crude coupling product ADC-2.1

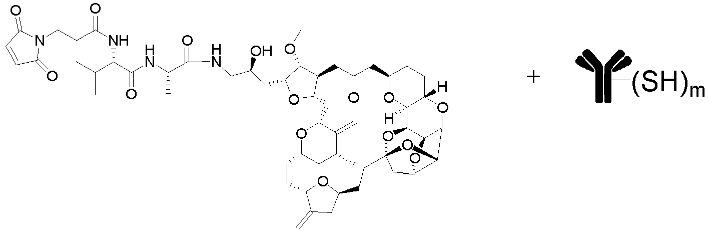

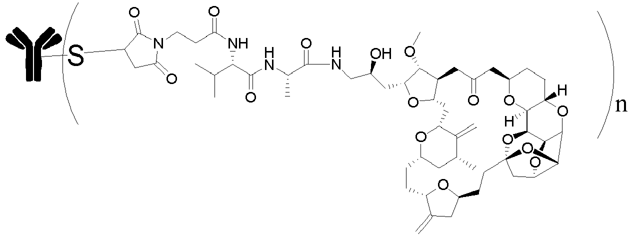

The antibody prepared in Example 1 was added to a pH 7.4 PBS solution at 10 mg/mL, stirred and mixed by using a 25°C water bath, and simultaneously added with an equal volume of TCEP solution in 2.4 times molar amount, and the solution was allowed to stand and react in a 25°C water bath for 1.5 hours. A 25°C water bath was used for the sample, then the antibody solution was stirred and mixed, and simultaneously added with Compound B in 8 times molar amount in DMSO solution (the final concentration of DMSO was 10%), the reaction was carried out under shaking in a shaker at 25°C for 90 minutes; finally, the sample was stirred and mixed, and simultaneously added with cysteine in 8 times molar amount, and allowed to stand and react in a 25°C water bath for 10 minutes to obtain the coupling product ADC-2.1.

### 2.1.3 Detection of crude coupling product ADC-2.1

The crude product of the coupling reaction was detected by SEC.

SEC chromatography conditions were as follows:
Column model: TSKgel G4000SWxl 7.8mmI.D.*30cm, 8µm
Detector wavelength: 280 nm/220 nm
Column temperature: 30°C
Flow rate: 1 mL/min
Elution mode: isocratic elution
Injection volume: 10 µL
Running time: 55 minutes

### 2.1.4 Purification of coupling reaction product:

After desalting and purification through PD-10 desalting column (packing: sephadex G 25), the conjugate was obtained. The conjugate was then exchanged into a solution of MES 25mM pH5.5 with 5% trehalose. SEC detection showed that small molecules had been completely removed. The detection result of purified ADC-2.1 was shown in Figure 2-1.

### 2.1.5 Determination of DAR

An equal volume of 50mM DTT solution was added to the ADC-2.1 and monoclonal antibody solutions, vortexed and mixed well in a 37°C water bath for 30 minutes. The reduced ADC and antibody were analyzed by RP-HPLC for conjugated and unconjugated antibody light and heavy chains. The DAR value was determined to be 4.1 by analyzing their compositions.

### 2-2. Preparation of conjugate ADC-2.2

### 2.2.1 Synthesis of crude coupling product ADC-2.2

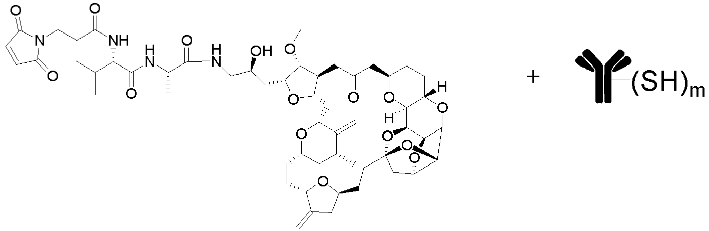

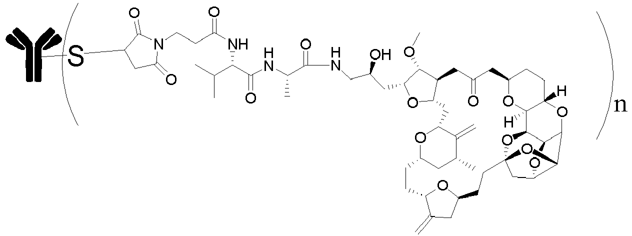

The antibody prepared in Example 1 was added to a pH 7.3 PBS solution at 10 mg/mL, stirred and mixed by using a 22°C water bath, and simultaneously added with an equal volume of TCEP solution in 2.15 times molar amount, and the solution was allowed to stand and react in a 22°C water bath for 2 hours. A 22°C water bath was used for the sample, then the antibody solution was stirred and mixed, and simultaneously added with Compound B in 5.5 times molar amount in DMSO solution (the final concentration of DMSO was 10%), the reaction was carried out under shaking in a shaker at 22°C for 60 minutes; finally, the sample was stirred and mixed, simultaneously added with cysteine in 8 times molar amount, and allowed to stand and react in a 22°C water bath for 10 minutes to obtain the coupling product ADC-2.2.

### 2.2.2 Detection of crude coupling product ADC-2.2

The crude product of the coupling reaction was detected by SEC.

SEC chromatography conditions were as follows:
Column model: TSKgel G4000SWxl 7.8mmI.D.*30cm, 8µm
Detector wavelength: 280 nm/220 nm
Column temperature: 30°C
Flow rate: 1 mL/min
Elution mode: isocratic elution
Injection volume: 10 µL
Running time: 55 minutes

### 2.2.3 Purification of coupling reaction product:

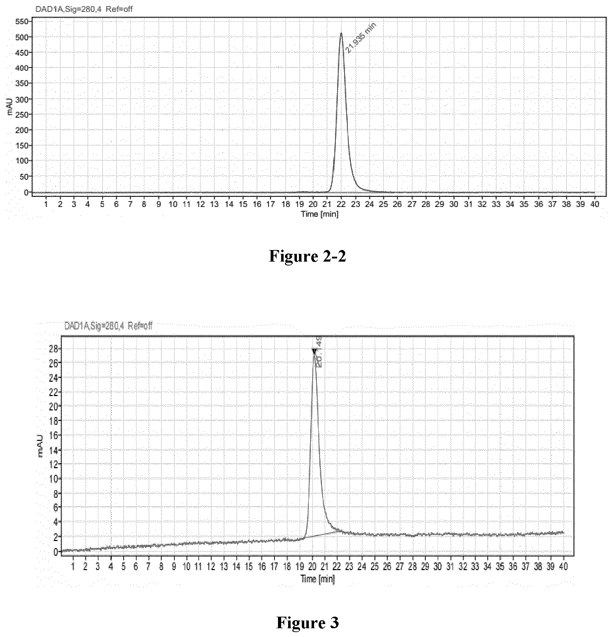

After desalting and purification through PD-10 desalting column (packing: sephadex G 25), the conjugate was obtained. The conjugate was then exchanged into a solution of His 20mM pH6.0 with 5% sucrose. SEC detection showed that the small molecules had been completely removed. The detection result of purified ADC-2.2 was shown in Figure 2-2.

### 2.2.4 Determination of DAR

An equal volume of 50mM DTT solution was added to the ADC-2.2 and monoclonal antibody solutions, vortexed and mixed well in a 37°C water bath for 30 minutes. The reduced ADC and antibody were analyzed by RP-HPLC for conjugated and unconjugated antibody light and heavy chains. The DAR value was determined to be 3.5 by analyzing their compositions.

### 3. Preparation of Control conjugate 1, ADC-4

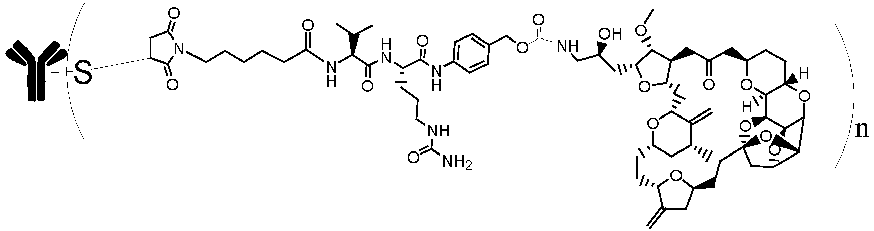

### 3.1 Preparation of intermediate C

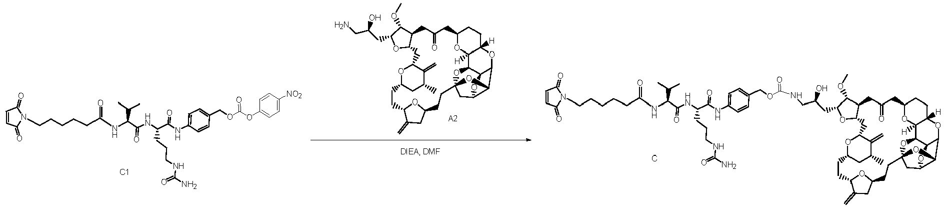

C1 (25 mg) and eribulin A2 (25 mg) were dissolved in DMF (3 mL), and added with DIEA (8.7 mg) at room temperature, and the reaction solution was stirred at room temperature for 2 hours. HPLC showed the formation of new peaks, and LCMS showed the production of target product. After Pre-HPLC purification and lyophilization, approximately 21 mg of the target compound C was obtained. LCMS: [M+1]+=1329.4.

### 3.2 Synthesis of crude coupling product ADC-4

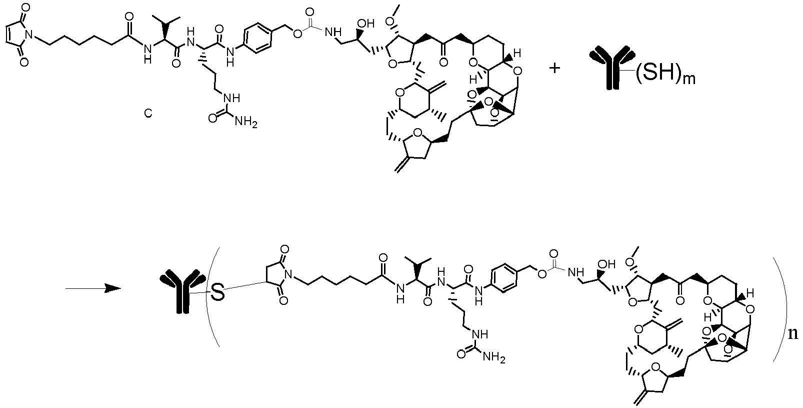

The antibody prepared in Example 1 was added in a pH 7.4 PBS solution at 10 mg/mL, and placed in an ice water bath. When the temperature of the protein solution reached 4°C, an equal volume of TCEP solution in 3.5 times molar amount was added while stirring and mixing. The solution was allowed to stand and react in a water bath at 25°C for 1 hour. A 25°C water bath was used for the reaction solution. When the temperature dropped to 25°C, the antibody solution was added with 40% DMSO solution of compound C in 7 times molar amount while stirring and mixing. The reaction was carried out under shaking on a shaker at 25°C for 90 minutes. Finally, the reaction solution was added with cysteine in 8 times molar amount while stirring and mixing, and allowed to stand and react in a water bath at 25°C for 10 minutes to obtain the coupling product ADC-4.

The detection, purification and DAR value determination methods of the coupling product were the same as those of ADC-1.1, and the DAR value of ADC-4 obtained was 3.8.

### 4. Preparation of Control conjugate 2, ADC-5

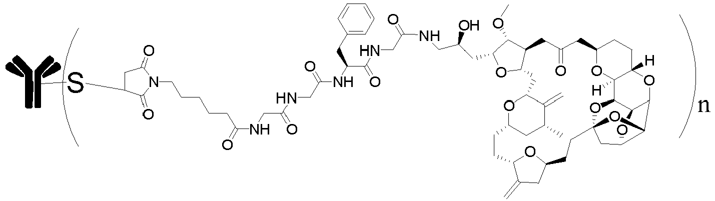

### 4.1 Preparation of Intermediate C

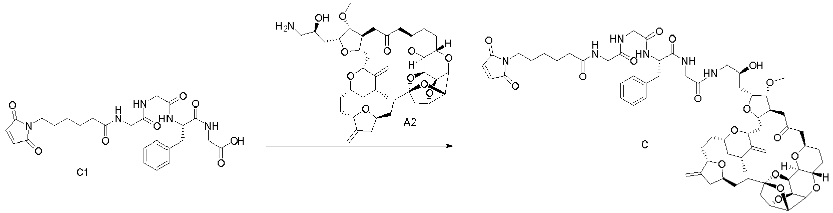

C1 (30 mg), eribulin A2 (30mg) and HATU were dissolved in DMF (0.5 mL), and added with DIEA (21 µl) at room temperature, the reaction solution was stirred at room temperature for 2 hours. HPLC showed the formation of new peaks, and LCMS showed the production of target product. After Pre-HPLC purification and lyophilization, approximately 25 mg of the target compound C was obtained. LCMS: [M+1]+=1241.6.

### 4.2 Synthesis of crude coupling product ADC-5

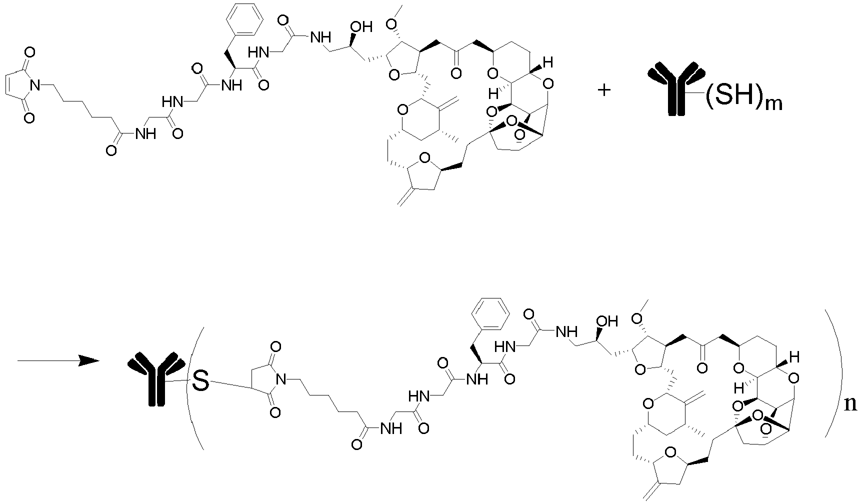

The antibody prepared in Example 1 was added to a pH 7.4 PBS solution at 5 mg/mL, and added with TCEP solution in 7 times molar amount while stirring and mixing, and the solution was allowed to stand and react in a 37°C water bath for 1.5 hours. A 25°C water bath was used for the reaction solution. When the temperature dropped to 25°C, the antibody solution was added with 40% DMSO solution of Compound C in 16 times molar amount while stirring and mixing. The reaction was carried out under shaking on a shaker at 25°C for 90 minutes. Finally, the reaction solution was added with cysteine in 16 times molar amount while stirring and mixing, and allowed to stand and react in a water bath at 25°C for 10 minutes to obtain the coupling product ADC-5.

### 4.3 Detection of crude coupling product ADC-5

The crude product of the coupling reaction was detected by SEC.

SEC chromatography conditions were as follows:
Column model: TSKgel G4000SWxl 7.8mmI.D.*30cm, 8µm
Detector wavelength: 280 nm/220 nm
Column temperature: 30°C
Flow rate: 1 mL/min
Elution mode: isocratic elution
Injection volume: 10 µL
Running time: 55 minutes

### 4.4 Purification of coupling reaction product:

After desalting and purification through PD-10 desalting column (packing: sephadex G 25), the conjugate was obtained. The conjugate was then exchanged into a solution of MES 25mM pH5.5 with 5% trehalose. SEC detection showed that small molecules had been completely removed. The SEC-HPLC result of purified ADC-5 was shown in Figure 3.

### 4.5 Determination of DAR

An equal volume of 50mM dithiothreitol (DTT) solution was added to the ADC-5 and monoclonal antibody solutions, vortexed and mixed well in a 37°C water bath for 30 minutes. The reduced ADC and antibody were analyzed by RP-HPLC for conjugated and unconjugated antibody light and heavy chains. The DAR value was determined to be 7.2 by analyzing their compositions.

### 5. Preparation of control conjugate ADC-217

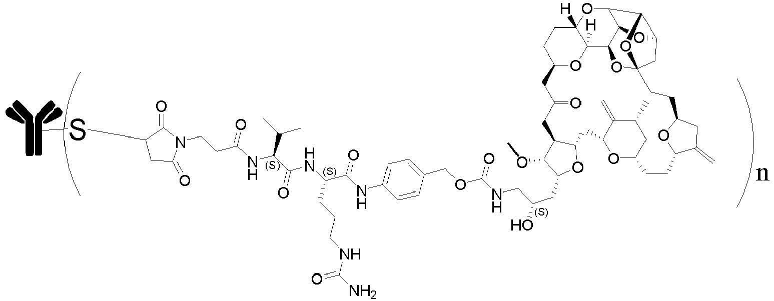

### 5.1 Synthesis of Fmoc-VC-PABC-eribulin

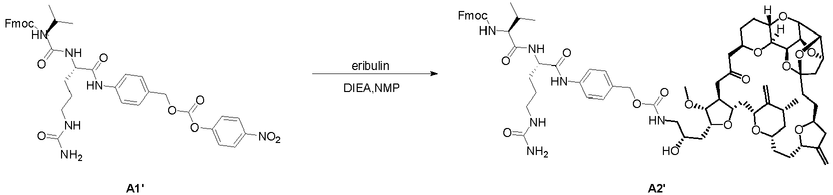

To a solution of A1' (50 mg, 0.065 mmol) in NMP (3 mL), eribulin (19.04 mg, 0.026 mmol) and DIEA (25.28 mg, 0.20 mmol) were added. The reaction solution was reacted at room temperature for 3 hours. The reaction solution was purified by Prep-HPLC and lyophilized to obtain about 71 mg of the target product A2'. LCMS: [M+1]+=1357.1.

### 5.2 Synthesis of VC-PABC-eribulin

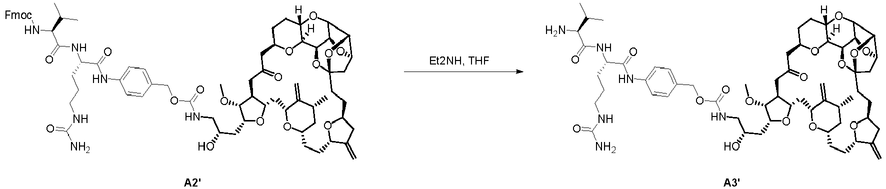

To a solution of A2' (71 mg, 0.052 mmol) in THF (3 mL), diethylamine (38.25 mg, 0.52 mmol) was added. The reaction solution was stirred and reacted at room temperature for 3 hours. The reaction solution was directly purified by Pre-HPLC and lyophilized to obtain about 48 mg of the target product A3'. LCMS: [M+1]+=1135.4.

### 5.3 Synthesis of MP-VC-PABC-eribulin

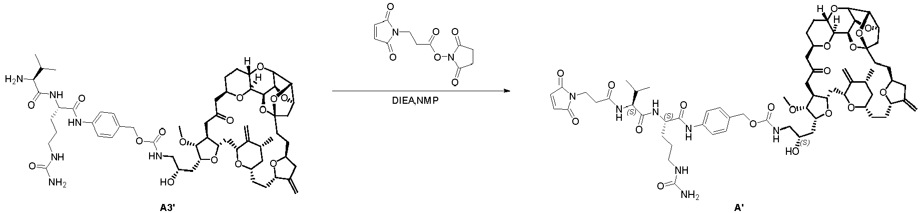

To a solution of A3' (20 mg, 0.018 mmol) in NMP (2 mL), DIEA (6.83 mg, 0.053 mmol) and MP-Osu (4.7 mg, 0.017 mmol) were added. The reaction solution was stirred and reacted at room temperature for 3 hours. The reaction solution was directly purified by Pre-HPLC to obtain about 16 mg of the target product A'. LCMS: [M+1]+=1386.4.

### 5.4 Conjugation of antibody:

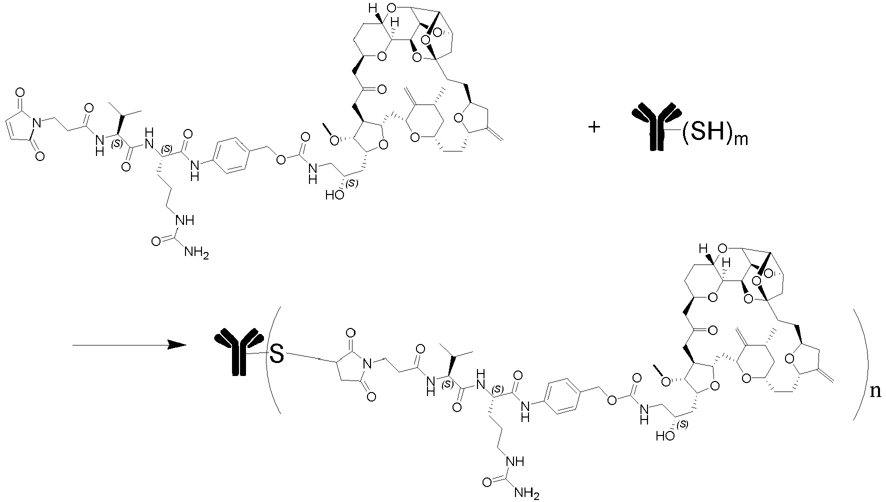

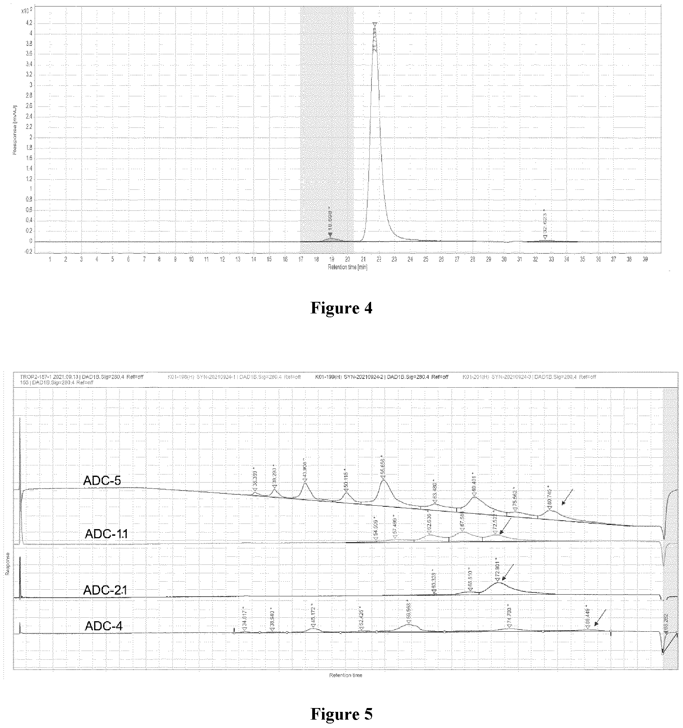

The TROP-2 antibody (20mM acetic acid-sodium acetate, pH6.1) prepared in Example 1 was adjusted to pH7.5 and diluted with a buffer of 50mM EPPS with 5mM EDTA at pH7.5. The product prepared in Section 5.3 was dissolved in DMSO. Protein solution (concentration 10mg/ml) (pH 7.5) was placed in a 25°C water bath. When the temperature of the protein solution reached 25°C, 1/10 volume of TCEP (dissolved in water) (2.2989eq) was added while stirring and mixing. The solution was reacted under shaking at 30 rpm on a shaker at 25°C for 90 minutes. A 25°C water bath was used for the sample, 5% DMSO was added while vortexing and mixing, then 5% Compound A' (7.5eq) was added, making the total DMSO 10%, and the reaction was carried out under shaking at 30 rpm on a shaker at 25°C for 90 minutes. The sample was added with Cys (dissolved in water) (8eq) while vortexing and mixing, and the reaction was carried out under shaking at 30 rpm on a shaker at 25°C for 10 minutes. The sample was centrifuged at 5000 rpm/min for 5 min, desalted with AKTA and exchanged into a solution of 25 mM MES, pH 5.5. SEC detection showed that small molecules had been completely removed. The detection result of purified ADC-217 was shown in Figure 4.

The drug-to-antibody ratio DAR was estimated to be 3.80 by RP-HPLC, and the detection method was the same as that of ADC-1.1.

### Example 3: Detection of hydrophilicity of various ADCs

A protein hydrophobic interaction chromatography column (HIC) was used to detect the hydrophilicity and hydrophobicity of various ADCs prepared in Example 2. The specific experimental conditions were as follows:

30 ul of each ADC sample prepared in Example 2 was taken and injected into HIC (TSKgel Butyl-NPR 4.6 mm I.D.×10cm,2.5um), the sample tray temperature was 4°C, the column temperature was 40°C. Mobile phase A: 1.5M Na₂SO₄&0.025M PB pH7.4&2%IPA (isopropyl alcohol), and mobile phase B: 0.025M PB pH7.4&11%IPA (isopropyl alcohol). The elution gradient was as follows:

| Elution gradient | Time (min) | A% | B% | Flow rate (ml/min) |
|---|---|---|---|---|
| 0.00 | | 100 | 0.0 | 0.600 |
| 15.00 | | 100 | 0.0 | 0.600 |
| 90.00 | | 7.4 | 92.6 | 0.600 |
| 95.00 | | 7.3 | 92.7 | 0.600 |
| 95.10 | | 100 | 0.0 | 0.600 |
| 100.00 | | 100 | 0.0 | 0.600 |

The results were shown in Figure 5. The positions indicated by arrows were the peak positions of various ADCs. The further left the peak position, the better the hydrophilicity. Comparing the hydrophilicity of the four ADCs in Example 2, it could be seen that the order of hydrophilicity was ADC-1.1≈ADC-2.1>ADC-5>ADC-4. Therefore, the L-D of the ADCs of the present disclosure (e.g., ADC-1.1 or ADC-2.1) has better hydrophilicity compared to the L-D of MORAb-202.

### Example 4: Affinity experiment between ADC and TROP protein

The affinity between each ADC prepared in Example 2 and TROP protein was detected to compare the affinity differences of ADCs with different structures.

### Detection method:

Coating Trop2 protein: Trop2 protein (Beijing Yiqiao Shenzhou Biotechnology Co., Ltd.) was diluted with coating solution (0.15M Na₂CO₃ and 0.35 M NaHCO₃) to 0.5µg/mL, mixed well and added to an ELISA plate, 100 µL/well, covered with sealing film, and placed in a 4°C refrigerator overnight.

Washing plate: The ELISA plate was taken out and washed 3 times with 1* PBST.

Blocking: Blocking solution (1% BSA) was taken and added to the plate, 200 µL/well of blocking solution, covered with sealing film, mixed at 250 rpm and incubated at 25°C for 1 hour.

Washing plate: The plate was washed 3 times with 1* PBST.

Incubating antibodies: The ADC samples 1, 2, 4, and 5 prepared in Example 2 and the TROP-2 antibody prepared in Example 1 were diluted to 2000ng/mL with 1% BSA, then they were subjected 3-fold dilution to 12 concentrations, and added to the ELISA plate, 100 µL/well, covered with sealing film, mixed at 250 rpm and incubated at 25°C for 1 hour.

Washing plate: The ELISA plate was washed 3 times with 1* PBST.

Incubating secondary antibody: Anti-Human HRP (Boster) was diluted with 1% BSA to 1: 10000, added at 100 µL/well to the ELISA plate, covered with sealing film, mixed at 250 rpm and incubated at 25°C for 1 hour.

Washing plate: The ELISA plate was washed 3 times with 1* PBST.

Color development: Color development substrate TMB (Huzhou Yingchuang) was added at 100ul/well to the ELISA plate, mixed well at 250 rpm and allowed to stand at 25°C for 15 minutes.

Stopping and reading: Stop solution (1M H₂SO₄) was added, 100ul/well, and a microplate reader was used for reading at OD450nm.

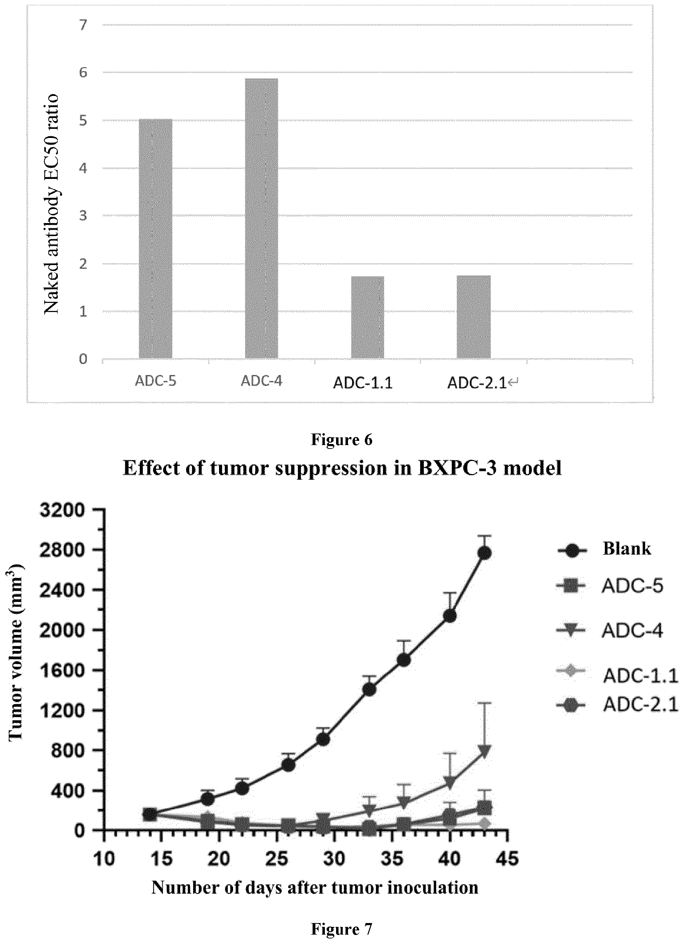

The detection results were shown in Figure 6. The ratio of the EC50 of the ADC prepared in Example 2 to the EC50 of the TROP-2 naked antibody is used as the ordinate. The larger the ratio, the smaller the affinity. Comparing the affinities of ADC products with different structures, the results showed that ADC-1.1 and ADC-2.1 had the best affinity, while the ADCs with a similar structure to MORAb-202 had the worst affinity with TROP-2. That was, the ADCs obtained by coupling the L-D structures of the present disclosure could better maintain the affinity of the antibody.

### Example 5: Anti-tumor effect of ADCs prepared in Example 2 on pancreatic cancer

### 1. Experimental methods

### 1.1 Cell culture

BxPC-3 cells (human orthotopic pancreatic adenocarcinoma cells, ATCC CRL-1687) were cultured in monolayer in vitro in an incubator at 37°C with air containing 5% CO₂ under culture conditions of: 1640 medium + 10% heat-inactivated fetal bovine serum + agar. Digestion with 0.25% trypsin was performed twice a week for passage. When the cells were in the exponential growth phase, the cells were collected, counted, and inoculated.

### 1.2 Tumor cell inoculation and tumor mass passage

5.0×10⁶ BxPC-3 tumor cells were suspended in a mixture of 0.1 ml PBS and Matrigel (1:1) and inoculated into the right scapula of 5 nude mice (P1 generation). When the tumor grew to 500 to 800 mm³, the tumor-bearing mice were killed by CO₂ anesthesia. The tumor masses were taken out, the surrounding necrotic tissues were removed, and the tumor masses were cut into small tumor masses of 20 to 30 mm³, and inoculated into a batch of new nude mice (P2 generation).

### 1.3 Tumor mass inoculation, grouping and administration

In this experiment, P6 generation tumor tissues were used to evaluate the anti-tumor activity of the samples to be tested. When the P5 tumor grew to 500 to 800 mm³, the tumor-bearing mice were killed by CO₂ anesthesia, the tumor masses were taken out, the surrounding necrotic tissues were removed, and the tumor masses were cut into small tumor masses of 20 to 30 mm³, and inoculated into the right scapula of the formal experiment mice, and a total of 40 mice were inoculated. When the average tumor volume reached about 135 mm³ 18 days after the tumor inoculation, mice with tumors that were too small or too large were eliminated, and the remaining 25 mice were randomly divided into groups according to tumor volume, and administered with drugs. In this experiment, the animals were administered only once, and no more administration was performed after the first administration on the day of grouping. The dosage regimen was shown in the following table.

| Group | Number of mice | Control/sample to be tested | Dose (mg/kg) | Route of administration |
|---|---|---|---|---|
| 1 | 5 | Blank (PBS) | -- | IV |
| 2 | 5 | ADC-1.1 | 0.02 | IV |
| 3 | 5 | ADC-2.1 | 0.02 | IV |
| 4 | 5 | ADC-4 | 0.02 | IV |
| 5 | 5 | ADC-5 | 0.02 | IV |

### 1.5 Experimental observation and data collection

After the tumor cells were inoculated, in addition to observing tumor growth, the effects of drug treatment on animal behavior were also monitored: the experimental animals' activity, food and water intake, body weight changes (body weight and tumor volume were measured twice a week), eyes, hair and other abnormalities. Clinical symptoms observed during the experiment were recorded in the raw data. The calculation method of tumor volume was: tumor volume (mm³) = 1/2 × (a × b²) (wherein, a represented the long diameter, and b represented the short diameter).

When the body weight of a single animal decreased by more than 15% (BWL>15%), the corresponding single animal was subjected to drug withdrawal treatment. Once the weight loss returned to within 10%, the drug administration would be resumed. When a single mouse lost >20% of its body weight, it would be euthanized in accordance with animal welfare.

### 1.6 Efficacy evaluation criteria

Relative tumor proliferation rate, T/C %, was a relative tumor volume or tumor weight percentage between the treatment group and the control group at a certain time point. The calculation formula was as follows: T/C % = T_{RTV} / C_{RTV} × 100% (T_{RTV}: average RTV of the treatment group; C_{RTV}: average RTV of the vehicle control group; RTV = Vₜ/V₀, V₀ represented tumor volume of the animal at the time of grouping, Vt represented tumor volume of the animal after treatment); or, T/C % = T_{TW} / C_{TW} × 100% (T_{TW}: average tumor weight of the treatment group at the end of experiment; C_{TW}: average tumor weight of the vehicle control group at the end of the experiment).

### 1.7 Statistical analysis

In this experiment, one-way ANOVA was used to compare tumor mean values among various groups. Homogeneity of variance was analyzed to show significant differences in F values. After ANOVA analysis, Dunnet's T3 (heterogeneity of variance) method was used for multiple comparisons. All data were analyzed by SPSS 17.0. p<0.05 was considered a significant difference.

### 2. Experimental results

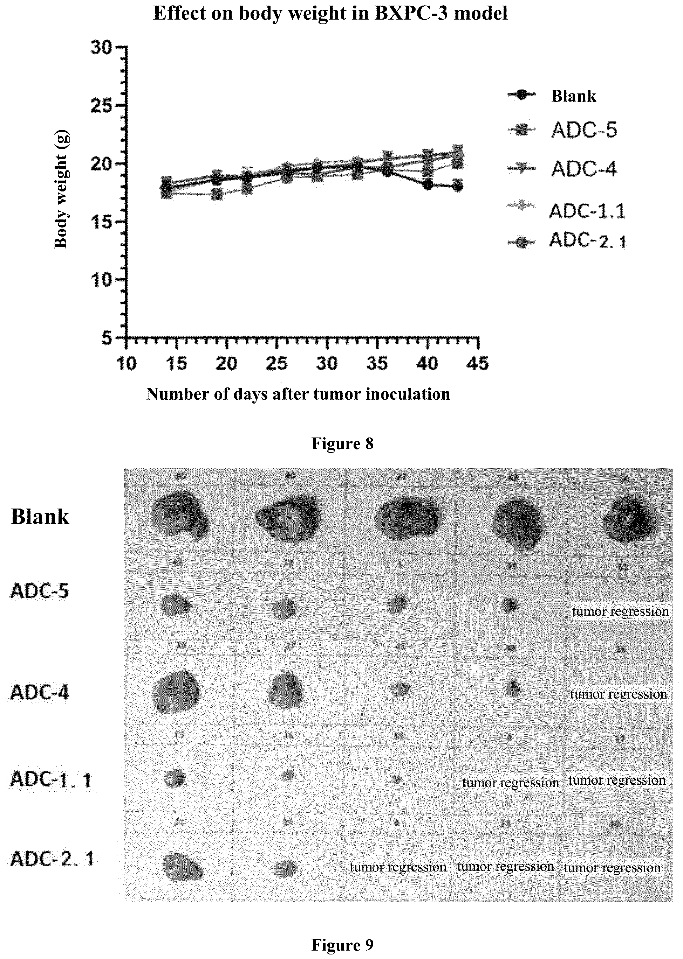

The experimental results were shown in Figures 7 to 9. Figure 7 showed the statistical curve of tumor inhibitory activity, Figure 8 showed the body weight change curve, and Figure 9 showed the photography of removed tumors.

It could be seen from the results that ADC-1.1 and ADC-2.1 had better anti-tumor effects than ADC-4 and ADC-5, wherein ADC-1.1 and ADC-2.1 could completely regress tumors with just a single administration and showed good safety.

### Example 6: Bystander effect of ADC --- Detection of bystander effect of ADC prepared in Example 2 (e.g., ADC-1.1)

Experimental design: Single cell culture group (A549-Luc) and mixed cell culture group (BxPC-3 and A549-Luc) were set. The ADC-1.1 prepared in Example 2 was used as the detection group, the hRS7 antibody prepared in Example 1 was used as the control group, a blank group was set to culture A549-Luc cells without adding drug, and a solvent control well was also set. BxPC-3 cells highly expressed TROP-2, and A549 cells lowly expressed TROP-2. The former could endocytose ADC-1.1 to a greater extent, while the latter had very low endocytosis efficiency. A549 was loaded with a fluorescent label. If the ADC-1.1 of the present disclosure had bystander effect, the eribulin small molecule toxin could be released by lysing BxPC-3 cells in the mixed cell culture group, thereby killing A549 cells, so that the fluorescence in the cells was quenched, and the absorbance value at 490nm read from a ELISA reader could be used to calculate the cell inhibition rate of the bystander effect.

### Experimental method:

1. BxPC-3 cells (human orthotopic pancreatic adenocarcinoma cells, ATCC CRL-1687) highly expressing TROP-2 were selected and cultured in monolayer in vitro. The well-growing cells were digested with trypsin, centrifuged, counted, resuspended in RPMI1640/F12-K+ 10% FBS, and adjusted to have a cell density of 8×10⁴ cells/ml.
2. Fluorescently labeled A549-Luc cells (human non-small cell lung cancer cells, ATCC CCL-185 cells) lowly expressing TROP-2 were selected and cultured in monolayer in vitro. The well-growing cells were digested with trypsin, centrifuged, counted, resuspended in RPMI1640/F12-K+ 10% FBS, and adjusted to have a cell density of 1×10⁴ cells/ml and 2×10⁴ cells/ml. The cell suspension of 1×10⁴ cells/ml was inoculated into single cell culture group of each plate, 100 µl/well, and 3 repeated wells were set.
3. Co-culture: The A549-Luc cell suspension with concentration of 2×10⁴ cells/ml and the BxPC-3 cells with concentration of 8×10⁴ cells/ml were mixed in a ratio of 1:1. After being mixed well, they were inoculated into co-culture wells of each culture plate, 100 µl/well, and 3 repeated wells were set.
4. Addition of drug: The cells were cultured in a carbon dioxide incubator for 24 hours. On the next day, according to the experimental design, the ADC-1.1 sample prepared in Example 2 and the hRS7 antibody prepared in Example 1 were diluted accordingly, and added at 100 µL/well to the cells, and the culture was continued in the carbon dioxide incubator for 96 hours.
5. After the culture was completed, ONE-GLO detection reagent was added at 100 µl/well to the cells, shaken at room temperature for 3 minutes in the dark, and the absorbance at 490 nm was measured using a Molecular SpectraMax M5 multifunctional ELISA reader. The cell activity of the blank group was taken as 100%. The sample signal values were used to calculate inhibition rate (%): killing rate (%) = (1 - signal value of sample well /average signal value of solvent control well) × 100. The obtained data were graphed and analyzed on GraphPad Prism9 to check the bystander efficiency.

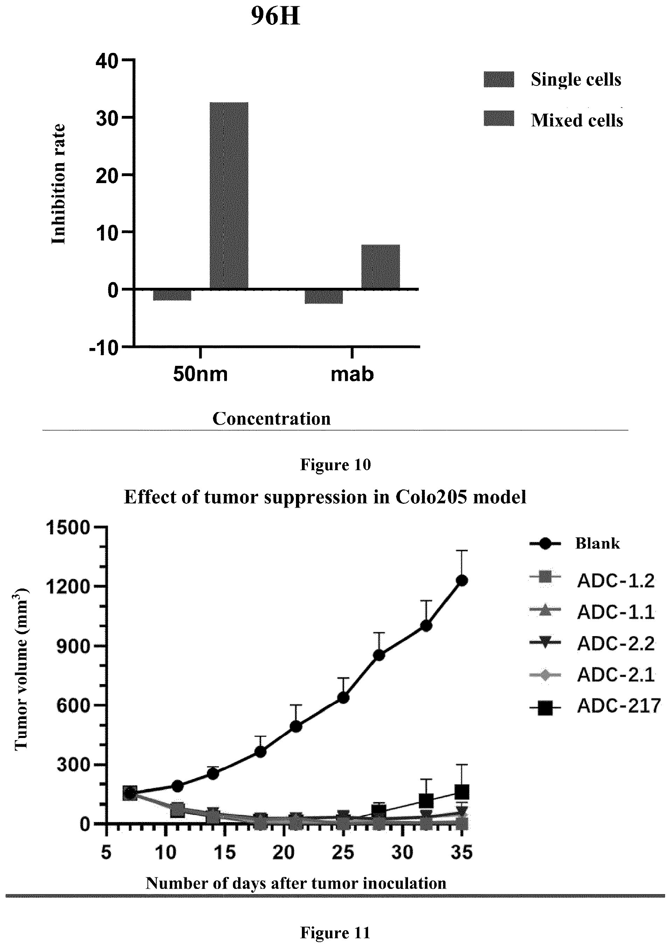

The detection results were shown in Figure 10. After ADC-1.1 was added to the cells, the mixed cells showed a cell inhibition rate of 30%, while the single cells did not show cell inhibition. TROP-2 naked antibody hRS7 also had an inhibition rate of 7% on the mixed cells, which was not as high as the inhibition rate of ADC-1.1. This result suggested that ADC-1.1 had bystander effect, and could kill not only target cells highly expressing TROP-2 in tumor cells, but also adjacent TROP-2-negative cells in a certain extent.

### Example 7: Tumor inhibitory effect of ADC prepared in Example 2 on colon cancer

### 1. Experimental method

### 1.1 Cell culture

Colo205 cells (human colon cancer cells, ATCC CCL-222) were cultured in monolayer in vitro in an incubator at 37°C with air containing 5% CO₂ under culture conditions of 1640 medium + 10% heat-inactivated fetal bovine serum + agar. Digestion with 0.25% trypsin was performed twice a week for passage. When the cells were in the exponential growth phase, the cells were collected, counted, and inoculated.

### 1.2 Tumor cell inoculation and tumor mass passage

5.0×10⁶ Colo205 tumor cells were suspended in a mixture of 0.1 ml PBS and Matrigel (1:1) and inoculated into the right scapula of 5 nude mice (P1 generation). When the tumor grew to 500 to 800 mm³, the tumor-bearing mice were killed by CO₂ anesthesia, the tumor masses were taken out, the surrounding necrotic tissues were removed, and the tumor masses were cut into small tumor masses of 20 to 30 mm³, and inoculated into a batch of new nude mice (P2 generation).

### 1.3 Tumor mass inoculation, grouping and administration

In this experiment, P6 generation tumor tissues were used to evaluate the anti-tumor activity of the samples to be tested. When the P5 tumor grew to 500 to 800 mm³, the tumor-bearing mice were killed by CO₂ anesthesia, the tumor masses were taken out, the surrounding necrotic tissues were removed, and the tumor masses were cut into small tumor masses of 20 to 30 mm³, and inoculated into the right scapula of the formal experimental mice, and a total of 50 mice were inoculated. Seven days after the tumor masses were inoculated, 30 mice with moderate tumor size were selected and randomly divided into groups according to tumor volume, and administered with drugs. The dosage regimen was as follows:

| Group | Number of mice | Control/sample to be tested | Dose (mg/kg) | Route of administration | Schedule of administration |
|---|---|---|---|---|---|
| 1 | 5 | Bland (PBS) | -- | IV | Only once |
| 2 | 5 | ADC-1.1 | 0.05 | IV | Only once |
| 3 | 5 | ADC-1.2 | 0.05 | IV | Only once |
| 4 | 5 | ADC-2.1 | 0.05 | IV | Only once |
| 5 | 5 | ADC-2.2 | 0.05 | IV | Only once |
| 6 | 5 | ADC-217 | 0.05 | IV | Only once |

| | | | | | |
|---|---|---|---|---|---|
| Note: "Only once" means no more administration after one administration on the day of grouping. | | | | | |

### 1.4 Experimental observation and data collection

After the tumor cells were inoculated, in addition to observing tumor growth, the effects of drug treatment on animal behavior were also monitored: the experimental animals' activity, food and water intake, weight changes (weight was measured twice a week), eyes, hire and others abnormal situations. Clinical symptoms observed during the experiment were recorded in the raw data. The calculation method of tumor volume was: tumor volume (mm³) = 1/2 × (a × b²) (wherein, a represented the long diameter, and b represented the short diameter).

When the body weight of a single animal decreased by more than 15% (BWL>15%), the corresponding single animal was subjected to drug withdrawal treatment. Once the weight loss returned to within 10%, the drug administration would be resumed. When a single mouse lost >20% of its body weight, it would be euthanized in accordance with animal welfare.

### 1.5 Efficacy evaluation criteria

Relative tumor proliferation rate, T/C %, was a relative tumor volume or tumor weight percentage between the treatment group and the control group at a certain time point. The calculation formula was as follows: T/C % = T_{RTV} / C_{RTV} × 100% (T_{RTV}: average RTV of the treatment group; C_{RTV}: average RTV of the vehicle control group; RTV = Vₜ/V₀, V₀ represented tumor volume of the animal at the time of grouping, Vt represented tumor volume of the animal after treatment); or, T/C % = T_{TW} / C_{TW} × 100% (T_{TW}: average tumor weight of the treatment group at the end of experiment; C_{TW}: average tumor weight of the vehicle control group at the end of the experiment).

### 1.6 Experimental endpoint

Four weeks after administration, tumors were removed from all mice, weighed, and photographed.

### 1.7 Statistical analysis

In this experiment, one-way ANOVA was used to compare tumor mean values among various groups. Homogeneity of variance was analyzed to show significant differences in F values. After ANOVA analysis, Dunnet's T3 (heterogeneity of variance) method was used for multiple comparisons. All data were analyzed by SPSS 17.0. p<0.05 was considered a significant difference.

### 2. Experimental results

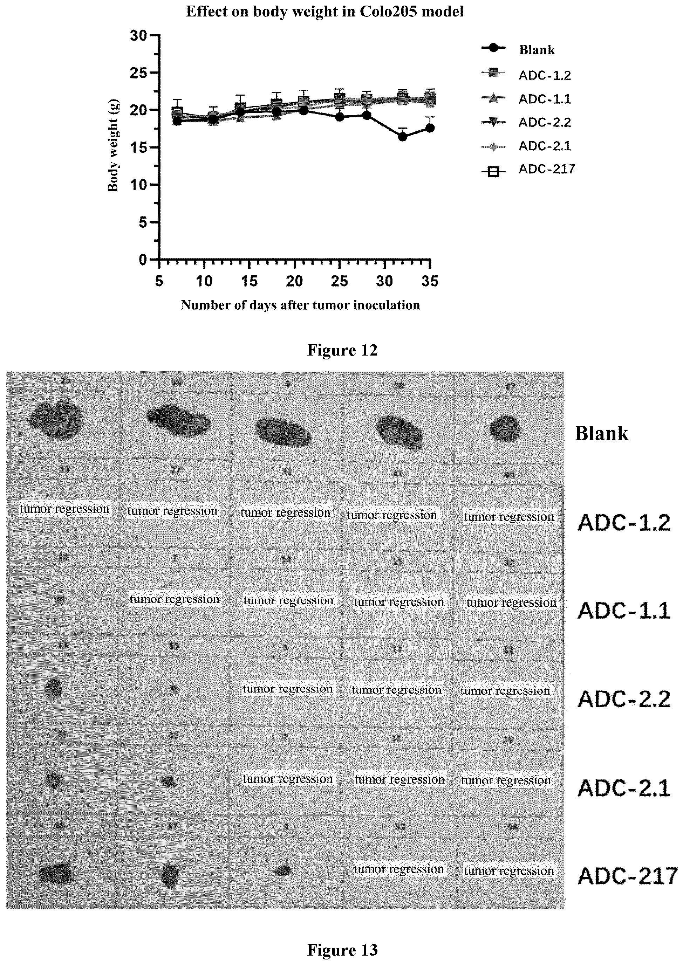

The experimental results were shown in the table below and Figures 11 to 13. Figure 11 showed the tumor inhibitory activity statistical curve, Figure 12 showed the weight change curve, and Figure 13 showed the photography of removed tumors. It could be seen from the results that the order of tumor inhibitory effects was: ADC-1.2≥ADC-1.1>ADC-2.1>ADC-2.2>>ADC-217 (control). Wherein, the tumors of all animals in the ADC-1.2 group completely regressed.

| Drug | N | Dose (mg/kg) | Body weight (g, mean±SEM) | | | Tumor volume/tumor weight (mm³/mg, mean±SEM) | | | | | | |
|---|---|---|---|---|---|---|---|---|---|---|---|---|
| | | | D7 | D35 | Change (%) | TV (mm³) | | TW (mg) | T/C (%) | | p value | |
| | | | | | | D7 | D35 | | TV | TW | TV | TW |
| Blank | 5 | - | 18.5±0.5 | 17.6±1.5 | -5.3 | 155±7 | 1232±150 | 968±138 | 100 | 100 | 1.000 | 1.000 |
| ADC-1.2 | 5 | 0.05 | 19.4±0.5 | 21.9±0.6 | +12.8 | 155±7 | 0±0 | 0±0 | 0 | 0 | .015 | .027 |
| ADC-1.1 | 5 | 0.05 | 18.6±0.5 | 21.0±0.5 | +12.9 | 155±6 | 12±12 | 5±5 | 1 | 0 | .015 | .027 |
| ADC-2.2 | 5 | 0.05 | 19.1±0.7 | 21.2±0.7 | +10.8 | 155±8 | 59±51 | 27±25 | 5 | 3 | .012 | .027 |
| ADC-2.1 | 5 | 0.05 | 18.9±0.3 | 21.3±0.1 | +12.9 | 155±8 | 47±35 | 23±17 | 4 | 2 | .014 | .028 |
| ADC-217 | 5 | 0.05 | 19.7±0.8 | 21.5±0.6 | +9.2 | 156±9 | 162±138 | 121±106 | 13 | 13 | .018 | .031 |

## Claims

1. A compound represented by Formula (I), a pharmaceutically acceptable salt thereof, a stereoisomer thereof, or a solvate thereof,
L¹-L²-D (I)
wherein:
L¹ is 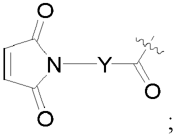
Yis 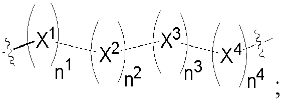
preferably, Y is 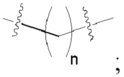
X¹, X², X³ and X⁴ are each independently selected from the group consisting of CH₂, NH, O and S, and at least one of X¹, X², X³ and X⁴ is CH₂;
preferably, X¹, X², X³ and X⁴ are all CH₂;
n¹, n², n³ and n⁴ are each independently selected from the group consisting of 0 and 1, and at least one of n¹, n², n³ and n⁴ is 1;
preferably, n¹ is 0, n², n³, and n⁴ are each independently selected from the group consisting of 0 and 1, and at least one of n², n³, and n⁴ is 1;
more preferably, n¹ and n² are 1, and n³ and n⁴ are 0;
n is selected from the group consisting of 1, 2, 3 and 4;
preferably, n is selected from the group consisting of 1, 2 and 3;
more preferably, n is 2;
L² is selected from the group consisting of amino acid residues and a short peptide consisting of 2 to 10 amino acid residues;
preferably, L² is a short peptide consisting of 2 to 4 amino acid residues, and preferably, the amino acid is selected from the group consisting of glycine, phenylalanine, valine, citrulline, and alanine, more preferably, the amino acid is selected from the group consisting of valine, citrulline, and alanine;
more preferably, L² is selected from the group consisting of glycine-glycine-phenylalanine-glycine, valine-citrulline, and valine-alanine, preferably selected from the group consisting of valine-citrulline and valine-alanine;
most preferably, L² is selected from the group consisting of 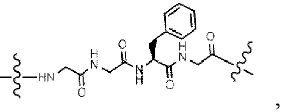 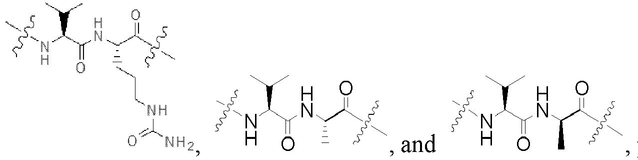 preferably selected from the group consisting of 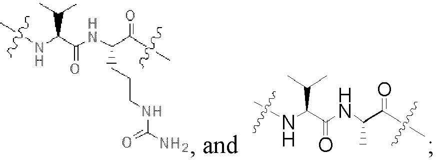
D is a drug linked to L² through a chemical bond, and the drug is preferably eribulin or a derivative thereof;
preferably, D is

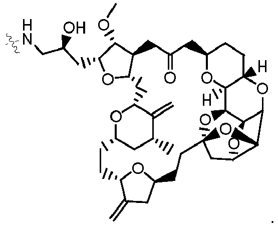

2. The compound, a pharmaceutically acceptable salt thereof, a stereoisomer thereof, or a solvate thereof according to claim 1, wherein the compound is selected from the group consisting of: 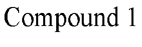 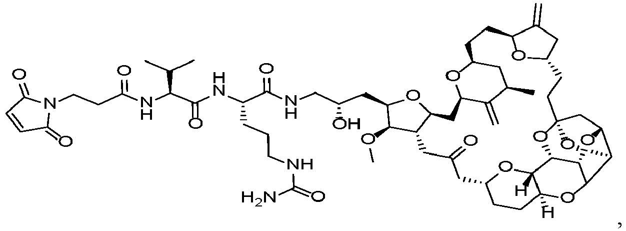 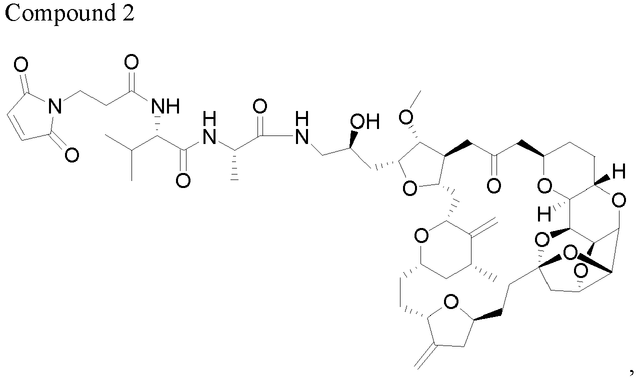 and

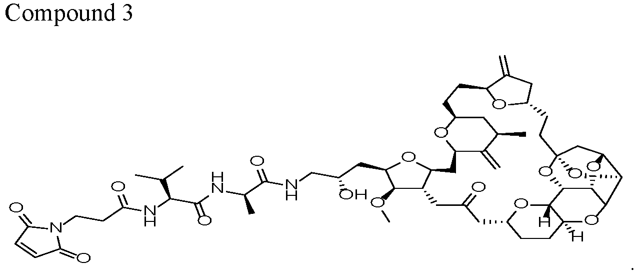

3. An antibody-drug conjugate represented by Formula (II), a pharmaceutically acceptable salt thereof, a stereoisomer thereof, or a solvate thereof,
Ab-(L¹-L²-D)ₚ (II)
wherein:
Ab represents an antibody or antigen-binding fragment thereof;
L¹, L² and D are as defined in claim 1;
p is any value between 1 and 20;
preferably, p is any value between 1 and 10;
more preferably, p is any value between 3 and 5;
most preferably, p is any value between 3.5 and 4.5, for example, p is 3.5, 3.9 or 4.1.

4. The antibody-drug conjugate, a pharmaceutically acceptable salt thereof, a stereoisomer thereof, or a solvate thereof according to claim 3, wherein the antibody is selected from the group consisting of anti-TROP-2 antibody, anti-her2 antibody, anti-her3 antibody, anti-claudin 18.2 antibody, anti-ROR-1 antibody, anti-dll-3 antibody, anti-muc1 antibody, and anti-muc-17 antibody; preferably, the antibody is a murine antibody, a chimeric antibody, or a humanized antibody; preferably, the humanized antibody is a fully human antibody;
or, the antigen-binding fragment is selected from the group consisting of Fab, Fab', F(ab')₂, single chain Fv (scFv), Fv and dsFv;
preferably, the antibody is an anti-TROP-2 antibody;
more preferably, the antibody is an anti-TROP-2 antibody, and the complementarity determining region (CDR) of the light chain variable region of the anti-Trop-2 antibody comprises CDR1 consisting of the amino acid sequence of KASQDVSIAVA, CDR2 consisting of the amino acid sequence SASYRYT, and CDR3 consisting of the amino acid sequence QQHYITPLT; the CDR of the heavy chain variable region comprises CDR1 consisting of the amino acid sequence NYGMN, CDR2 consisting of the amino acid sequence WINTYTGEPTYTDDFKG, and CDR3 consisting of the amino acid sequence GGFGSSYWYFDV; preferably, the amino acid sequences of the light chain and heavy chain of the anti-Trop-2 antibody are shown in SEQ ID NO: 1 and SEQ ID NO: 2, respectively; preferably, the nucleotide sequences coding the light chain and heavy chain of the anti-Trop-2 antibody are shown in SEQ ID NO:3 and SEQ ID NO:4, respectively.

5. The antibody-drug conjugate, a pharmaceutically acceptable salt thereof, a stereoisomer thereof, or a solvate thereof according to any one of claims 3 to 4, wherein the antibody-drug conjugate is selected from the group consisting of: 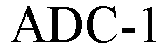 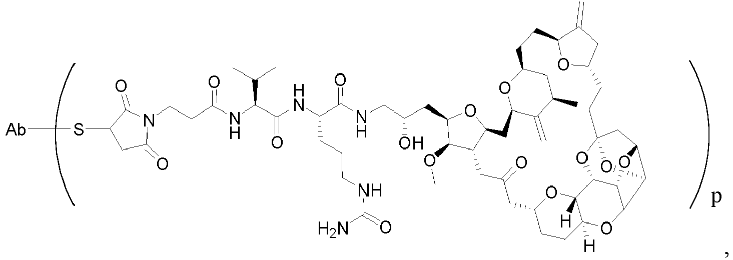 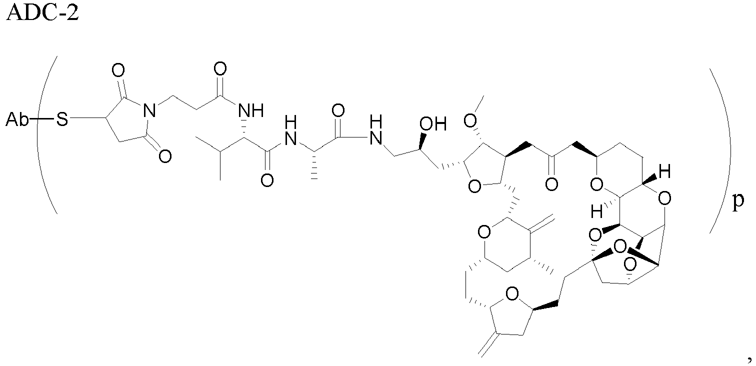 and

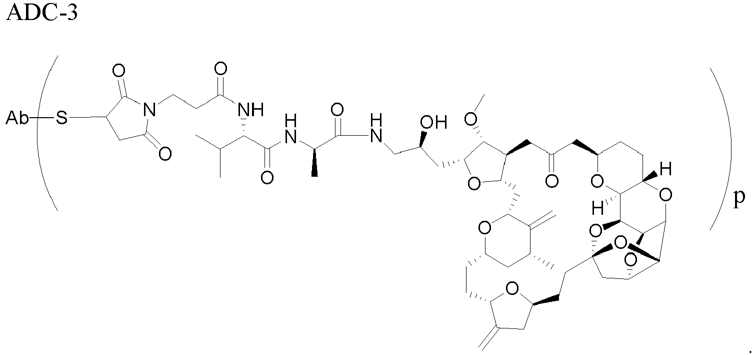

6. A method for preparing the compound represented by Formula (I), a pharmaceutically acceptable salt thereof, a stereoisomer thereof, or a solvate thereof according to any one of claims 1 to 2, which comprises:
performing an amidation reaction between L¹-L²-OH and eribulin to obtain the compound represented by Formula (I), a pharmaceutically acceptable salt thereof, a stereoisomer thereof, or a solvate thereof, wherein, L¹, L² are as defined in claim 1.

7. A method for preparing the antibody-drug conjugate represented by Formula (II), a pharmaceutically acceptable salt thereof, a stereoisomer thereof, or a solvate thereof according to any one of claims 3 to 5, which comprises:
performing a reaction between the compound represented by Formula (I), a pharmaceutically acceptable salt thereof, a stereoisomer thereof, or a solvate thereof according to any one of claims 1 to 2 and the Ab as defined in any one of claims 3 to 4 to obtain the antibody-drug conjugate represented by Formula (II), a pharmaceutically acceptable salt thereof, a stereoisomer thereof, or a solvate thereof;
preferably, the compound represented by Formula (I), a pharmaceutically acceptable salt thereof, a stereoisomer thereof, or a solvate thereof according to any one of claims 1 to 2 is obtained by the method according to claim 6.

8. A pharmaceutical composition, which comprises the compound represented by Formula (I), a pharmaceutically acceptable salt thereof, a stereoisomer thereof, or a solvate thereof according to any one of claims 1 to 2, or the antibody-drug conjugate represented by Formula (II), a pharmaceutically acceptable salt thereof, a stereoisomer thereof, or a solvate thereof according to any one of claims 3 to 5; optionally, further comprises one or more pharmaceutical adjuvants, such as carriers and/or excipients.

9. Use of the compound represented by Formula (I), a pharmaceutically acceptable salt thereof, a stereoisomer thereof, or a solvate thereof according to any one of claims 1 to 2, or the antibody-drug conjugate represented by Formula (II), a pharmaceutically acceptable salt thereof, a stereoisomer thereof, or a solvate thereof according to any one of claims 3 to 5, or the pharmaceutical composition according to claim 8 in the manufacture of a medicament for preventing and/or treating a disease associated with abnormal cell activity (e.g., a cancer disease);
preferably, the cancer is in-situ cancer or metastatic cancer;
preferably, the cancer is selected from the group consisting of metastatic breast cancer, non-small cell lung cancer, Burkitt lymphoma, Hodgkin's lymphoma, acute myeloid leukemia, chronic myeloid leukemia, acute lymphoblastic leukemia, chronic lymphoblastic leukemia, skin cancer, oral cancer, esophageal cancer, gastrointestinal cancer, lung tract cancer, lung cancer, gastric cancer, colon cancer, rectal cancer, triple negative breast cancer (TNBC), ovarian cancer, prostate cancer, uterine cancer, endometrial cancer, cervical cancer, bladder cancer, pancreatic cancer, bone cancer, brain cancer, connective tissue cancer, thyroid cancer, liver cancer, gallbladder cancer, bladder (urothelial) cancer, kidney cancer, skin cancer, central nervous system cancer, testicular cancer, epithelial cancer, and head and neck cancer;
more preferably, the cancer is selected from the group consisting of metastatic breast cancer, TNBC, non-TNBC, cervical cancer, endometrial cancer, lung cancer, ovarian cancer, bladder (urothelial) cancer, colon cancer, and pancreatic cancer;
most preferably, the cancer is selected from the group consisting of TNBC, ovarian cancer, cervical cancer, bladder (urothelial) cancer, and pancreatic cancer (e.g., in-situ pancreatic cancer).

10. Use of the compound represented by Formula (I), a pharmaceutically acceptable salt thereof, a stereoisomer thereof, or a solvate thereof according to any one of claims 1 to 2, or the antibody-drug conjugate represented by Formula (II), a pharmaceutically acceptable salt thereof, a stereoisomer thereof, or a solvate thereof according to any one of claims 3 to 5, or the pharmaceutical composition according to claim 8 in the manufacture of a reagent for inhibiting the growth, proliferation or migration of a cancer cell;
preferably, the cancer cell is in-situ cancer cell or metastatic cancer cell;
preferably, the cancer cell is a cancer cell of the cancer selected from the group consisting of metastatic breast cancer, non-small cell lung cancer, Burkitt lymphoma, Hodgkin's lymphoma, acute myeloid leukemia, chronic myeloid leukemia, acute lymphoblastic leukemia, chronic lymphoblastic leukemia, skin cancer, oral cancer, esophageal cancer, gastrointestinal cancer, lung tract cancer, lung cancer, gastric cancer, colon cancer, rectal cancer, triple negative breast cancer (TNBC), ovarian cancer, prostate cancer, uterine cancer, endometrial cancer, cervical cancer, bladder cancer, pancreatic cancer, bone cancer, brain cancer, connective tissue cancer, thyroid cancer, liver cancer, gallbladder cancer, bladder (urothelial) cancer, kidney cancer, skin cancer, central nervous system cancer, testicular cancer, epithelial cancer, and head and neck cancer;
more preferably, the cancer cell is a cancer cell of the cancer selected from the group consisting of metastatic breast cancer, TNBC, non-TNBC, cervical cancer, endometrial cancer, lung cancer, ovarian cancer, bladder (urothelial) cancer, colon cancer, and pancreatic cancer;
most preferably, the cancer cell is a cancer cell of the cancer selected from the group consisting of TNBC, ovarian cancer, cervical cancer, bladder (urothelial) cancer, and pancreatic cancer (e.g., a cancer cell of in-situ pancreatic cancer).
